# EUROPEAN PATENT APPLICATION

(11) **EP 1 736 172 A1**
(43) Date of publication of application: **27.12.2006**
(21) Application number: 05719492.0
(22) Date of filing: 24.02.2005
(51) Int. Cl.: A61K 45/00, A01K 67/027, A61K 31/138, A61K 31/7088, A61K 31/713, A61K 39/395, A61K 48/00, A61P 25/18, A61P 25/22, A61P 25/24, A61P 25/36, C12Q 1/02, C12Q 1/68, G01N 33/15, G01N 33/50, C07K 14/47, C07K 16/18, C12N 15/00

(54) **METHOD OF UTILIZING ORGANIC CATION TRANSPORTER OCT3-RELATED MOLECULE FOR TREATING MENTAL DISEASES SUCH AS DEPRESSION, ANXIETY NEUROSIS, DRUG ADDICTION AND THE LIKE**

(30) Priority: 09.03.2004 JP 2004065051
(71) Applicant: Biostation Inc., Tajimi-shi Gifu 5070015 (JP)
(72) Inventor: KITAICHI, Kiyoyuki, 5070015 (JP)
(74) Representative: Bohmann, Armin K.
(86) International application number: PCT/JP2005/003042
(87) International publication number: WO 2005/084707

(57) **Abstract**

Mice in which OCT3 expression is suppressed were successfully constructed by administering antisense against OCT3 gene into the brain. Mice in which OCT3 expression is suppressed display phenotypes related to mental disorders such as anti-depression and anti-anxiety, and therefore the mice can be applied to the screening of therapeutic agents for mental disorders. In addition, substances suppressing the OCT3 expression or function were shown to be in fact effective as therapeutic agents for depression and anxiety neuroses.

SEQUENCE LISTING

<110>  BIOSTATION INC.

<120>  METHOD OF UTILIZING ORGANIC CATION TRANSPORTER OCT3-RELATED MOLECULE FOR
TREATING MENTAL DISEASES SUCH AS DEPRESSION, ANXIETY NEUROSIS, DRUG ADDICTION
AND THE LIKE

<130>  B 10054 EP

<150>  JP 2004-065051
<151>  2004-03-09

<160>  7

<170>  PatentIn version 3.1

<210>  1
<211>  5624
<212>  DNA
<213>  Homo sapiens

<220>
<221>  CDS
<222>  (28)..(1698)
<223>

<400>  1
ctgcgggcgg cgggcggcgg gcgcacc atg ccc tcc ttc gac gag gcg ctg cag      54
                              Met Pro Ser Phe Asp Glu Ala Leu Gln
                              1               5

cgg gtg ggc gag ttc ggg cgc ttc cag agg cgc gtg ttt ttg ctg ctg      102
Arg Val Gly Glu Phe Gly Arg Phe Gln Arg Arg Val Phe Leu Leu Leu
10                  15                  20                  25

tgc ctg acg ggc gtc acc ttc gcc ttc ctc ttc gtc ggc gtg gtc ttc      150
Cys Leu Thr Gly Val Thr Phe Ala Phe Leu Phe Val Gly Val Val Phe
                30                  35                  40

ctg ggc acg cag ccc gac cac tac tgg tgc cgc ggg cca agt gcc gcg      198
Leu Gly Thr Gln Pro Asp His Tyr Trp Cys Arg Gly Pro Ser Ala Ala
            45                  50                  55

gcg ctg gcc gag cgc tgc ggc tgg agc ccg gag gag gag tgg aac cgc      246
Ala Leu Ala Glu Arg Cys Gly Trp Ser Pro Glu Glu Glu Trp Asn Arg
        60                  65                  70

acg gcg ccc gcc tcc cgc ggc cca gag ccc ccc gag cgc cgc ggc cgc      294
Thr Ala Pro Ala Ser Arg Gly Pro Glu Pro Pro Glu Arg Arg Gly Arg
    75                  80                  85

tgc cag cgc tac ctc ctg gag gcg gcc aac gac agc gcc tcc gcc act      342
Cys Gln Arg Tyr Leu Leu Glu Ala Ala Asn Asp Ser Ala Ser Ala Thr
90                  95                  100                 105

agc gct ctc agc tgc gcg gac cca ctc gcc gcc ttc ccc aac cgt tcg      390
Ser Ala Leu Ser Cys Ala Asp Pro Leu Ala Ala Phe Pro Asn Arg Ser
                110                 115                 120

gct ccc ctt gtg ccg tgc cgc ggc ggc tgg cgc tac gcc cag gcc cac      438
Ala Pro Leu Val Pro Cys Arg Gly Gly Trp Arg Tyr Ala Gln Ala His
            125                 130                 135

tcc acc atc gtc agc gag ttt gac ctt gtc tgt gtc aat gcg tgg atg 486
Ser Thr Ile Val Ser Glu Phe Asp Leu Val Cys Val Asn Ala Trp Met
        140                 145                 150

ctg gac ctc acc caa gcc atc ctg aac ctc ggc ttc ctg act gga gca 534
Leu Asp Leu Thr Gln Ala Ile Leu Asn Leu Gly Phe Leu Thr Gly Ala
    155                 160                 165

ttc acc tta ggc tat gca gca gac agg tat ggc agg atc gtc att tac 582
Phe Thr Leu Gly Tyr Ala Ala Asp Arg Tyr Gly Arg Ile Val Ile Tyr
170                 175                 180                 185

ttg cta tcc tgc ctt ggt gtt ggc gtc act ggg gtt gtg gtg gcc ttt 630
Leu Leu Ser Cys Leu Gly Val Gly Val Thr Gly Val Val Val Ala Phe
                190                 195                 200

gca cca aac ttc cct gtg ttt gtg atc ttc cgc ttc ctg caa ggt gta 678
Ala Pro Asn Phe Pro Val Phe Val Ile Phe Arg Phe Leu Gln Gly Val
            205                 210                 215

ttt gga aag ggg acg tgg atg act tgc tac gtg att gtg aca gaa ata 726
Phe Gly Lys Gly Thr Trp Met Thr Cys Tyr Val Ile Val Thr Glu Ile
        220                 225                 230

gta ggt tcg aaa caa agg agg att gtg gga atc gtg att caa atg ttc 774
Val Gly Ser Lys Gln Arg Arg Ile Val Gly Ile Val Ile Gln Met Phe
    235                 240                 245

ttt acc ctt gga atc ata att ctc cct gga att gcc tac ttc atc ccc 822
Phe Thr Leu Gly Ile Ile Ile Leu Pro Gly Ile Ala Tyr Phe Ile Pro
250                 255                 260                 265

aac tgg caa gga atc cag tta gcc atc acg ctg ccc agc ttt ctc ttc 870
Asn Trp Gln Gly Ile Gln Leu Ala Ile Thr Leu Pro Ser Phe Leu Phe
                270                 275                 280

ctc ctt tat tac tgg gtg gtc cct gag tct ccc cgt tgg ctg att act 918
Leu Leu Tyr Tyr Trp Val Val Pro Glu Ser Pro Arg Trp Leu Ile Thr
            285                 290                 295

cgg aag aaa gga gat aaa gca tta cag atc ctg aga cgc att gct aag 966
Arg Lys Lys Gly Asp Lys Ala Leu Gln Ile Leu Arg Arg Ile Ala Lys
        300                 305                 310

tgc aat ggg aaa tac ctc tca tca aat tac tca gag atc act gtt aca 1014
Cys Asn Gly Lys Tyr Leu Ser Ser Asn Tyr Ser Glu Ile Thr Val Thr
    315                 320                 325

gat gag gaa gtt agt aat cca tcc ttt tta gat ctg gtg aga act ccc 1062
Asp Glu Glu Val Ser Asn Pro Ser Phe Leu Asp Leu Val Arg Thr Pro
330                 335                 340                 345

caa atg agg aaa tgc aca ctt att ctt atg ttt gct tgg ttc aca agc 1110
Gln Met Arg Lys Cys Thr Leu Ile Leu Met Phe Ala Trp Phe Thr Ser
                350                 355                 360

gca gtg gtg tat caa gga ctt gtc atg cgc ctg gga att ata ggg ggc 1158
Ala Val Val Tyr Gln Gly Leu Val Met Arg Leu Gly Ile Ile Gly Gly
            365                 370                 375

aac ctc tat ata gac ttt ttc atc tcg ggc gtg gtg gaa ctg cca gga 1206
Asn Leu Tyr Ile Asp Phe Phe Ile Ser Gly Val Val Glu Leu Pro Gly

        380                 385                 390

gct ctc ttg atc tta cta acc att gag cgc ctt gga cga cgc ctc ccc 1254
Ala Leu Leu Ile Leu Leu Thr Ile Glu Arg Leu Gly Arg Arg Leu Pro
    395                 400                 405

ttt gcg gca agc aat ata gtg gca ggg gtg gca tgc ctt gtc act gcg 1302
Phe Ala Ala Ser Asn Ile Val Ala Gly Val Ala Cys Leu Val Thr Ala
410                 415                 420                 425

ttc tta cca gaa gga ata gca tgg ttg agg acc aca gtg gct aca ttg 1350
Phe Leu Pro Glu Gly Ile Ala Trp Leu Arg Thr Thr Val Ala Thr Leu
                430                 435                 440

gga aga cta ggg ata acc atg gcc ttt gaa att gtt tat ttg gta aat 1398
Gly Arg Leu Gly Ile Thr Met Ala Phe Glu Ile Val Tyr Leu Val Asn
            445                 450                 455

tca gaa ttg tac cca aca aca tta cga aat ttc gga gtt tcg ctc tgt 1446
Ser Glu Leu Tyr Pro Thr Thr Leu Arg Asn Phe Gly Val Ser Leu Cys
        460                 465                 470

tca ggt ctg tgt gat ttt ggg gga atc ata gcc cca ttt ctg ctc ttt 1494
Ser Gly Leu Cys Asp Phe Gly Gly Ile Ile Ala Pro Phe Leu Leu Phe
    475                 480                 485

cgg cta gca gcc gtg tgg cta gaa cta cct ctg atc atc ttt ggt atc 1542
Arg Leu Ala Ala Val Trp Leu Glu Leu Pro Leu Ile Ile Phe Gly Ile
490                 495                 500                 505

ctg gca tcc atc tgt ggt ggc ctt gtg atg ctt ttg cct gaa acc aag 1590
Leu Ala Ser Ile Cys Gly Gly Leu Val Met Leu Leu Pro Glu Thr Lys
                510                 515                 520

ggt att gcc ttg cca gag aca gtg gat gat gta gaa aaa ctt ggc agt 1638
Gly Ile Ala Leu Pro Glu Thr Val Asp Asp Val Glu Lys Leu Gly Ser
            525                 530                 535

cca cat tcc tgt aaa tgt ggc agg aat aag aaa acc cca gtt tcc cgc 1686
Pro His Ser Cys Lys Cys Gly Arg Asn Lys Lys Thr Pro Val Ser Arg
        540                 545                 550

tct cac ctt tga ggcccccgac aaagacagaa agaaggagct atccaggagc 1738
Ser His Leu
    555

tgatcctcct tgcaaagctg tgccttgcag agatgcacgt gtgcatttca gctacatcat 1798

gccgcgctgt tgtaatactg tataaagacc tcaatctatc cagagtattt ttatataatg 1858

ttggatgagt taggatttgt aatgctgttg aagtttctgg gaacacataa tatgtagcca 1918

gtttaacaaa gaagctgtca ggtgcacagc ccttcctggg tttttttctt gtgttccctg 1978

tggtctctga cccattaggc taaagagaga caagagaagc ccccaacctg attctcatga 2038

cagctccatc aagaatgtgg gatgtgccga ccaaggattt gagaaagttg tacagaaatg 2098

tgttcatcaa atctggtcaa gggactaagc tcctagctga ccattcattc tgaagattgc 2158

atggaggatg aacatctggg aatcctgtta atgagaaggc tgaatcacag gcacctgggc 2218

caaagggtgt gagcattcat gttctctgct caccttggtt tccgcacacc ttcgcaatgt 2278

gaacaggtca ggagtccctc ccgtccacct cctctgtaac agctggggtt ccaggcatgg 2338

tttaggccct gttccagcaa taagaaccaa tctgctgtac aatctgagga cttggctgtg 2398

ttatttacaa aatgatgctg tggttctgag attatttggg acatttttgg ctctccttta 2458

gtggacacct agagccacag attcccttct ttactaaaca aatcccatgg attctgattt 2518

ctgggtctta ggattttaaa agtgaaggga tatttttctt atatttgtga gttcagttcc 2578

gatggtgccc gtggtcaaaa gcgaaaaaca tggacaattc ctattcattc ttagcacttt 2638

gacatgtctt ggggaaaagc ttacatttta atttaaaaga aagatcaatt atatccatgc 2698

ttaacaggat cagcaggagc tttataaatg actttacaga gactaataag ggatttgatc 2758

tttctttttt tgttatcgag gcttttgaaa tgtggaactt gtgtgttctg ctttatatgt 2818

tatattcaat atcttttcag atgcagtcta tattttatgc tgagttttaa aaatgaaata 2878

ctttatgcaa acaggcaaaa ttggtaccaa agggaaacat taaccatgag gaagagcatt 2938

tttctaagga gaacaggtga caatatacac atgtgcgcta atcgtaaaat gagcatctta 2998

gtctttaaaa cacatcagaa ttgaatacga ataatctatt tgtcgatgaa ataaacacaa 3058

ctctttgagg atttgagact acattcaccc tttattcaca gtcacttgca gttttgcttt 3118

tctctgcatt tctctgctgt aagatgactg ttgcattgtt gaattgtatt ttgagtggat 3178

atttttgttt ggtaacaatt aaaattttaa atcgtaaaaa atgtacattt gccctttctc 3238

ttccttctaa acatgtgtct ctcccacagg tcattctcct gtgacacgtg tactgcagag 3298

tgtttcaaaa ccgggcaggc aattagcaat gggaaataag tggtccacct tcaatatttg 3358

cttgttgttc atttacatgc tcttggcacc aatcctagac tcacgtgtgc cccagaataa 3418

cattcagact ctcagctggt cttgtgttac acatccatgg accggttcac tccatcatat 3478

acagctctct gctccgtgtc ccctgggctc aagtcaagca gtcggtgaca gatttcattc 3538

ccaataacag aatcggtttg catgactccc catacatgtt gcagctttga aaacattcat 3598

ctcagagtta ggtataaaga cataaaaatg tgtgtcaagc cctcgttagc tgatgaggta 3658

aatgcatgga caacttccta ggacttctcg gctctgccct ctgtgacctt gcagcctgct 3718

caggtctttg tgctctgttc cttatcaaaa acagcacaac cccctcaatc tgcccattta 3778

aaaattggtg gagtgaacaa agagcatgaa aatgttgaag accacaacca aaatttactc 3838

aaatcaaagt taagtacaga gtttattttt aacaaatgtc atagctatgt caccttacta 3898

cagaatccag cagtcagaaa ataacacaaa aaatgccgag gtttggcgta gctggtagct 3958

ggggacagtg tcttcgtttg attactgcca gttatttcca gcatgctaaa tccctaccca 4018

cgttccagcc tctaggtgag tcagtgcgtc actctgtctc ccgtccaatt aattatttct 4078

catcactccc tcaatccaag taacaaacct tgaaacacga acatagacac caggcttatt 4138

ggggcgtgca cagccaagac cccaagaagt gactccttgt aaaatgtatt tgtccttctc 4198

aaagcaaacc agaggccctc cactgtcacc ctaaatagag gtagggaaaa attcatgtga 4258

gggttaggct gccagccttt tattgtggca tccatctatt gtccatatgt atattttccc 4318

agtaattgaa attggaaagg aacttatggg catattactg gagcatctgc agtgcttaag 4378

tacttagcaa aatctattca gcgaaaccac accacttagt ttctcccagg ggtaaaaaaa 4438

acccttgtga atcctgcaca catcccttcc tgtgttggtg tttcctttag actggcctag 4498

cctgggcatc actgtaaagt atgtgcagtt ggtctctgtc ctgtaccctc tacctccttc 4558

cctgtcggaa aagtcaccac tttagtccct ggccaccagc acaccccagg agggtgagtg 4618

gcctgaggta gttacggcac ttaaaactcc cttgctaccg atctggaact caagccccaa 4678

gacatcccct tagatgatct gaatacgcat tcagggacag atctaggcag tttctaaaca 4738

acacttagac tggggtctaa cgttgacaaa tccttctaga atttgcctct ttgggactga 4798

agtctaaggg gctgagacca agaagggaga gcacaagact aactttggtc tcttgacctt 4858

ttcttacctt gcaactgtca gtgcctccag ccaaatgcct agcacagagc tcagtggatt 4918

tgattcttcc attcagatac tcatagcgat tgcacacttt attctcaatc acaagaagct 4978

ggggcttcct tgagaaggcc agccccaaag gtacctgtgt ttaaaagatg aaagaaatgg 5038

ttgctgaacc caaaacatgt ttaggaggaa aaccagaaga acagagcagt ggcactggac 5098

ctcctgtctg tccgtgaggc tgcagaacac aaggcagctc ccaatgccgc tggactctct 5158

gaccttggag agtgtaaaca tcatatatga ttctgtgttc ctgagatctg ggtttaacga 5218

tctgggcttt gtcatggtgt tttacgttcg ttgttgtttg tgtacttttt gcttttcttt 5278

tgaaagtatg aaaatgattt ttacaatgta aagagagtct gagaaatgca attgctattc 5338

tttttatata ctttttcaga ttttttatat gcttctttac tacttacatc ataatgcaca 5398

attttatata ctgatttttt caatcaatgt atatcatgag tttttaaaat aatgctacaa 5458

tcttcataac taatatttta atggggagtg actgcttaat tggcatgagg tttccttttg 5518

agatgaggaa aatgttctga aaatatggtg atggttgcac accattgtga atgtactgaa 5578

tgccattcag tggtaccctg caaaataatt aaaatcctaa ctctta 5624

<210> 2
<211> 556
<212> PRT
<213> Homo sapiens

<400> 2
Met Pro Ser Phe Asp Glu Ala Leu Gln Arg Val Gly Glu Phe Gly Arg
1               5                   10                  15

Phe Gln Arg Arg Val Phe Leu Leu Leu Cys Leu Thr Gly Val Thr Phe
            20                  25                  30

Ala Phe Leu Phe Val Gly Val Val Phe Leu Gly Thr Gln Pro Asp His
        35                  40                  45

Tyr Trp Cys Arg Gly Pro Ser Ala Ala Ala Leu Ala Glu Arg Cys Gly
    50                  55                  60

Trp Ser Pro Glu Glu Glu Trp Asn Arg Thr Ala Pro Ala Ser Arg Gly
65                  70                  75                  80

Pro Glu Pro Pro Glu Arg Arg Gly Arg Cys Gln Arg Tyr Leu Leu Glu
                85                  90                  95

Ala Ala Asn Asp Ser Ala Ser Ala Thr Ser Ala Leu Ser Cys Ala Asp
            100                 105                 110

Pro Leu Ala Ala Phe Pro Asn Arg Ser Ala Pro Leu Val Pro Cys Arg
        115                 120                 125

Gly Gly Trp Arg Tyr Ala Gln Ala His Ser Thr Ile Val Ser Glu Phe
    130                 135                 140

Asp Leu Val Cys Val Asn Ala Trp Met Leu Asp Leu Thr Gln Ala Ile
145                 150                 155                 160

Leu Asn Leu Gly Phe Leu Thr Gly Ala Phe Thr Leu Gly Tyr Ala Ala
                165                 170                 175

Asp Arg Tyr Gly Arg Ile Val Ile Tyr Leu Leu Ser Cys Leu Gly Val
            180                 185                 190

Gly Val Thr Gly Val Val Val Ala Phe Ala Pro Asn Phe Pro Val Phe
        195                 200                 205

Val Ile Phe Arg Phe Leu Gln Gly Val Phe Gly Lys Gly Thr Trp Met
    210                 215                 220

Thr Cys Tyr Val Ile Val Thr Glu Ile Val Gly Ser Lys Gln Arg Arg
225                 230                 235                 240

Ile Val Gly Ile Val Ile Gln Met Phe Phe Thr Leu Gly Ile Ile Ile
                245                 250                 255

Leu Pro Gly Ile Ala Tyr Phe Ile Pro Asn Trp Gln Gly Ile Gln Leu
            260                 265                 270

Ala Ile Thr Leu Pro Ser Phe Leu Phe Leu Leu Tyr Tyr Trp Val Val
        275                 280                 285

Pro Glu Ser Pro Arg Trp Leu Ile Thr Arg Lys Lys Gly Asp Lys Ala
    290                 295                 300

Leu Gln Ile Leu Arg Arg Ile Ala Lys Cys Asn Gly Lys Tyr Leu Ser
305                 310                 315                 320

Ser Asn Tyr Ser Glu Ile Thr Val Thr Asp Glu Glu Val Ser Asn Pro
                325                 330                 335

Ser Phe Leu Asp Leu Val Arg Thr Pro Gln Met Arg Lys Cys Thr Leu
            340                 345                 350

Ile Leu Met Phe Ala Trp Phe Thr Ser Ala Val Val Tyr Gln Gly Leu
        355                 360                 365

Val Met Arg Leu Gly Ile Ile Gly Gly Asn Leu Tyr Ile Asp Phe Phe
    370                 375                 380

Ile Ser Gly Val Val Glu Leu Pro Gly Ala Leu Leu Ile Leu Leu Thr
385                 390                 395                 400

Ile Glu Arg Leu Gly Arg Arg Leu Pro Phe Ala Ala Ser Asn Ile Val
                405                 410                 415

Ala Gly Val Ala Cys Leu Val Thr Ala Phe Leu Pro Glu Gly Ile Ala
            420                 425                 430

Trp Leu Arg Thr Thr Val Ala Thr Leu Gly Arg Leu Gly Ile Thr Met
        435                 440                 445

Ala Phe Glu Ile Val Tyr Leu Val Asn Ser Glu Leu Tyr Pro Thr Thr
    450                 455                 460

Leu Arg Asn Phe Gly Val Ser Leu Cys Ser Gly Leu Cys Asp Phe Gly
465                 470                 475                 480

Gly Ile Ile Ala Pro Phe Leu Leu Phe Arg Leu Ala Ala Val Trp Leu
                485                 490                 495

Glu Leu Pro Leu Ile Ile Phe Gly Ile Leu Ala Ser Ile Cys Gly Gly
            500                 505                 510

Leu Val Met Leu Leu Pro Glu Thr Lys Gly Ile Ala Leu Pro Glu Thr
        515                 520                 525

Val Asp Asp Val Glu Lys Leu Gly Ser Pro His Ser Cys Lys Cys Gly
    530                 535                 540

Arg Asn Lys Lys Thr Pro Val Ser Arg Ser His Leu
545                 550                 555

<210>  3
<211>  3499
<212>  DNA
<213>  Mus musculus

<220>
<221>  CDS
<222>  (381)..(2036)
<223>

<400>  3
ttgggcccct gcagcctaag agtactggag gctattcctg gagtcttcag agtcctgagt      60

ccagcgagtc tgggtgatcc tggaactcag tgaactctga cttggggtgt ccctttaggg     120

caggctacag gtcagggccc ttggtgcaca gtgacactac tgtgaaacca ccccaatccg     180

ctctccgggt ttactccggt cccgggcgcg cacgggcagg aggccgggag cttggaacgg     240

gaggagcagc ccggggcggg ctgccagggc gagcaagcga gcgaggcggg ggcggcgggt     300

gcagagctac gggcggcggc gcccgcgggt cactctcagg ccgggcagca gcgacagggc     360

gcagggtagc agggcgcacc atg ccc acg ttc gac cag gca ctg agg aag gcg     413
                      Met Pro Thr Phe Asp Gln Ala Leu Arg Lys Ala
                      1               5                   10

ggc gag ttc ggg cgc ttc cag cgg cgc gtg ttc ctg ctg ctg tgc ctg 461
Gly Glu Phe Gly Arg Phe Gln Arg Arg Val Phe Leu Leu Leu Cys Leu
            15                  20                  25

acg ggt gtc acc ttc gcc ttc ctc ttc gtc ggt gtg gtc ttc ctg ggc 509
Thr Gly Val Thr Phe Ala Phe Leu Phe Val Gly Val Val Phe Leu Gly
        30                  35                  40

agc cag ccc gac tac tat tgg tgt cgc ggg ccg cgc gcc acc gcg ctg 557
Ser Gln Pro Asp Tyr Tyr Trp Cys Arg Gly Pro Arg Ala Thr Ala Leu
    45                  50                  55

gcc gag cgc tgc gcc tgg agc ccc gag gag gag tgg aac ctc acc aca 605
Ala Glu Arg Cys Ala Trp Ser Pro Glu Glu Glu Trp Asn Leu Thr Thr
60                  65                  70                  75

ccg gag ctc cac gtc cca gct gag cgc cgc ggc caa ggc cac tgc cac 653
Pro Glu Leu His Val Pro Ala Glu Arg Arg Gly Gln Gly His Cys His
                80                  85                  90

cgc tac ctg ctg gaa gcc act aat acc agc tca gag ctc agc tgc gac 701
Arg Tyr Leu Leu Glu Ala Thr Asn Thr Ser Ser Glu Leu Ser Cys Asp
            95                  100                 105

cca ctc act gcc ttc ccc aac cgc tcc gcg cct ctg gtg tcc tgc agc 749
Pro Leu Thr Ala Phe Pro Asn Arg Ser Ala Pro Leu Val Ser Cys Ser
        110                 115                 120

ggt gac tgg cgc tat gtg gag acc cac tct acc atc gtc agc cag ttt 797
Gly Asp Trp Arg Tyr Val Glu Thr His Ser Thr Ile Val Ser Gln Phe
    125                 130                 135

gac ctt gtc tgc agc aat gcc tgg atg ttg gac ctc acc caa gcc atc 845
Asp Leu Val Cys Ser Asn Ala Trp Met Leu Asp Leu Thr Gln Ala Ile
140                 145                 150                 155

ctg aac ctt ggc ttc ctg gct ggg gct ttt acc ttg ggc tat gca gcg 893
Leu Asn Leu Gly Phe Leu Ala Gly Ala Phe Thr Leu Gly Tyr Ala Ala
                160                 165                 170

gac aga tat ggc agg ctc atc att tac tta ata tcc tgt ttc ggc gtt 941
Asp Arg Tyr Gly Arg Leu Ile Ile Tyr Leu Ile Ser Cys Phe Gly Val
            175                 180                 185

ggc atc aca gga gtc gtg gtg gca ttt gcg cca aat ttt tct gtg ttt 989
Gly Ile Thr Gly Val Val Val Ala Phe Ala Pro Asn Phe Ser Val Phe
        190                 195                 200

gtg att ttc cgc ttc cta caa gga gtg ttt gga aag ggg gcc tgg atg 1037
Val Ile Phe Arg Phe Leu Gln Gly Val Phe Gly Lys Gly Ala Trp Met
    205                 210                 215

act tgc ttc gtg atc gtg aca gaa ata gtt ggt tca aaa caa agg agg 1085
Thr Cys Phe Val Ile Val Thr Glu Ile Val Gly Ser Lys Gln Arg Arg
220                 225                 230                 235

att gtg gga atc gtg atc cag atg ttc ttc acc ctc ggg atc att att 1133
Ile Val Gly Ile Val Ile Gln Met Phe Phe Thr Leu Gly Ile Ile Ile
                240                 245                 250

ctt cct ggg att gcc tac ttc acg ccc agc tgg cag ggc atc cag cta 1181
Leu Pro Gly Ile Ala Tyr Phe Thr Pro Ser Trp Gln Gly Ile Gln Leu

            255                 260                 265

gcc atc tct ctg ccc agc ttt ctc ttc ctc ctc tat tac tgg gtg gtc 1229
Ala Ile Ser Leu Pro Ser Phe Leu Phe Leu Leu Tyr Tyr Trp Val Val
        270                 275                 280

cct gag tct ccc cgc tgg ctg atc acc cgg aag caa gga gag aaa gcc 1277
Pro Glu Ser Pro Arg Trp Leu Ile Thr Arg Lys Gln Gly Glu Lys Ala
    285                 290                 295

ttg cag atc ctg agg cgc gtg gct aag tgc aat gga aaa cac ctc tca 1325
Leu Gln Ile Leu Arg Arg Val Ala Lys Cys Asn Gly Lys His Leu Ser
300                 305                 310                 315

tca aat tac tca gag atc aca gtt aca gat gaa gaa gtc agt aac cca 1373
Ser Asn Tyr Ser Glu Ile Thr Val Thr Asp Glu Glu Val Ser Asn Pro
                320                 325                 330

tcc tgt tta gac ctt gtg aga act ccc caa atg agg aaa tgc acg ctc 1421
Ser Cys Leu Asp Leu Val Arg Thr Pro Gln Met Arg Lys Cys Thr Leu
            335                 340                 345

atc ctt atg ttt gct tgg ttc acg agc gcc gtg gtg tac caa gga ctt 1469
Ile Leu Met Phe Ala Trp Phe Thr Ser Ala Val Val Tyr Gln Gly Leu
        350                 355                 360

gtc atg cgc ctg gga ctt atc gga ggc aac ctc tac ata gac ttc ttt 1517
Val Met Arg Leu Gly Leu Ile Gly Gly Asn Leu Tyr Ile Asp Phe Phe
    365                 370                 375

atc tct ggg ctc gtg gag ctg ccc gga gct ctc tta atc ctt ctg acc 1565
Ile Ser Gly Leu Val Glu Leu Pro Gly Ala Leu Leu Ile Leu Leu Thr
380                 385                 390                 395

att gag cgc ctt gga cga cgc ctt ccc ttt gcg gca agc aat ata gtg 1613
Ile Glu Arg Leu Gly Arg Arg Leu Pro Phe Ala Ala Ser Asn Ile Val
                400                 405                 410

gca ggg gtg tcg tgt ctg gtc act gca ttt tta cca gaa ggg ata ccg 1661
Ala Gly Val Ser Cys Leu Val Thr Ala Phe Leu Pro Glu Gly Ile Pro
            415                 420                 425

tgg ctg agg acc aca gtc gct acc ctg gga aga cta ggg ata acc atg 1709
Trp Leu Arg Thr Thr Val Ala Thr Leu Gly Arg Leu Gly Ile Thr Met
        430                 435                 440

gcc ttc gaa att gtt tat ttg gta aat tca gag ttg tac cca acg aca 1757
Ala Phe Glu Ile Val Tyr Leu Val Asn Ser Glu Leu Tyr Pro Thr Thr
    445                 450                 455

tta cgg aac ttt ggg gtt tca ctc tgc tca ggc ttg tgt gac ttt ggg 1805
Leu Arg Asn Phe Gly Val Ser Leu Cys Ser Gly Leu Cys Asp Phe Gly
460                 465                 470                 475

ggg att ata gcc ccg ttt ctg ctc ttt cgg ctg gca gct ata tgg tta 1853
Gly Ile Ile Ala Pro Phe Leu Leu Phe Arg Leu Ala Ala Ile Trp Leu
                480                 485                 490

gaa ctg cct ctg atc atc ttt ggg atc ctg gcg tct gtc tgt ggt ggc 1901
Glu Leu Pro Leu Ile Ile Phe Gly Ile Leu Ala Ser Val Cys Gly Gly
            495                 500                 505

ctc gtg atg ctt ttg cct gaa acg aag ggc att gcc ttg ccg gag acg 1949

Leu Val Met Leu Leu Pro Glu Thr Lys Gly Ile Ala Leu Pro Glu Thr
        510                 515                 520

gtg gaa gac gta gaa aag ctt ggc agt tca cag ttg cat cag tgt ggc      1997
Val Glu Asp Val Glu Lys Leu Gly Ser Ser Gln Leu His Gln Cys Gly
    525                 530                 535

aga aaa aag aaa acc cag gtt tct act tct gat gtc tga ggcccctgtg      2046
Arg Lys Lys Lys Thr Gln Val Ser Thr Ser Asp Val
540                 545                 550

ttgcctggag gaccagagaa gactggaata tgcctgtgca gtgtgcttcg cctcctctag      2106

gtggcgccct tccaaggaac cgtaaagatt tcggagtatt ttataaggta taagatgaag      2166

atttatcatg ctgcccaagc ttcggggaac acataatctt ggacctgttt catgaagacc      2226

cagtaggtac ccggattctg gctaggttct cattcagcaa gtctctcacc cattaggtaa      2286

ggagagatga aaaggcccca accagttctc cagttctcag gacaagacag taccatccag      2346

gaagcaggat gaacaaacca aggcctggca cgtgtccaga agtgcgctca gctgatctga      2406

tcaacacacc gcagtgaaga gtgttctgaa gagtctgaga actgctattt cttcccttga      2466

gctccgaata caagccttcg cagggccgac atgtccggcg ctgctcccct cctgtctcct      2526

gcaggtgctg aggtcccagg tcccaggtta ggcacagctc cttaaatgca acacaacaga      2586

ggacctggct gtggtattgt gaagtcatgc tgcagtcccc aggccccaga tatttcgcca      2646

cctccttcag aggaccactg taatcacagg ctctttattg aggaagaaaa caaagcacgg      2706

atttggattt cccaggtctt agaatttaaa aagtaaaggg ataattatgt catatttgtg      2766

aattccgtgt ccccgggtgc ctgtgactga aagtccaaag cacagacttt tttcattcca      2826

tcttcacatt tttgacttgt cttgggagga aaaacaaaac aaaaaacaaa accaatacat      2886

ttgttgccac ttaatgataa aagtcagttg tattcaaact taaaagaatt agcagccaat      2946

ttgacttgta agtgttggag gagatactaa tgaggaactg gacttttcct ttctttcttt      3006

tttttttttt taatcaagcc ttctggagca gagactcaaa tgtaaacaca gtattctata      3066

tggtgtgcag ttcttggttt attttcaaac tcggtatgta tgttaggctg aactgtgtct      3126

ttaaacttca aactcagtgt ggacaggcaa aattagcatc aaagagaact tgccaatgtg      3186

tgtgtgtggg gggggaggaa ctatttcact tgtggagagc atactatgtt tgcaacactg      3246

gagaagatgt gtgtgactgt tagacagatg tttaatcata aagcacagga gaacggagtg      3306

tgatgctcag ttccccaaca gcctttgggg tattcattgc cacacccact gtgtacacag      3366

gcgtttgtgg cccggctgcc tgcgtgtttc gtggctgtag cacggctctt gtccaccctg      3426

ctgcctttgg attgcatttc aagttggtag ttctgtttca taacaataaa cgatttttaa      3486

actgttttta aaa                                                        3499

<210>  4

<211>  551
<212>  PRT
<213>  Mus musculus

<400>  4
Met Pro Thr Phe Asp Gln Ala Leu Arg Lys Ala Gly Glu Phe Gly Arg
1               5                   10                  15

Phe Gln Arg Arg Val Phe Leu Leu Leu Cys Leu Thr Gly Val Thr Phe
            20                  25                  30

Ala Phe Leu Phe Val Gly Val Val Phe Leu Gly Ser Gln Pro Asp Tyr
        35                  40                  45

Tyr Trp Cys Arg Gly Pro Arg Ala Thr Ala Leu Ala Glu Arg Cys Ala
    50                  55                  60

Trp Ser Pro Glu Glu Glu Trp Asn Leu Thr Thr Pro Glu Leu His Val
65                  70                  75                  80

Pro Ala Glu Arg Arg Gly Gln Gly His Cys His Arg Tyr Leu Leu Glu
                85                  90                  95

Ala Thr Asn Thr Ser Ser Glu Leu Ser Cys Asp Pro Leu Thr Ala Phe
            100                 105                 110

Pro Asn Arg Ser Ala Pro Leu Val Ser Cys Ser Gly Asp Trp Arg Tyr
        115                 120                 125

Val Glu Thr His Ser Thr Ile Val Ser Gln Phe Asp Leu Val Cys Ser
    130                 135                 140

Asn Ala Trp Met Leu Asp Leu Thr Gln Ala Ile Leu Asn Leu Gly Phe
145                 150                 155                 160

Leu Ala Gly Ala Phe Thr Leu Gly Tyr Ala Ala Asp Arg Tyr Gly Arg
                165                 170                 175

Leu Ile Ile Tyr Leu Ile Ser Cys Phe Gly Val Gly Ile Thr Gly Val
            180                 185                 190

Val Val Ala Phe Ala Pro Asn Phe Ser Val Phe Val Ile Phe Arg Phe
        195                 200                 205

Leu Gln Gly Val Phe Gly Lys Gly Ala Trp Met Thr Cys Phe Val Ile
    210                 215                 220

Val Thr Glu Ile Val Gly Ser Lys Gln Arg Arg Ile Val Gly Ile Val
225                 230                 235                 240

Ile Gln Met Phe Phe Thr Leu Gly Ile Ile Ile Leu Pro Gly Ile Ala
                245                 250                 255

Tyr Phe Thr Pro Ser Trp Gln Gly Ile Gln Leu Ala Ile Ser Leu Pro
            260                 265                 270

Ser Phe Leu Phe Leu Leu Tyr Tyr Trp Val Val Pro Glu Ser Pro Arg
        275                 280                 285

Trp Leu Ile Thr Arg Lys Gln Gly Glu Lys Ala Leu Gln Ile Leu Arg
    290                 295                 300

Arg Val Ala Lys Cys Asn Gly Lys His Leu Ser Ser Asn Tyr Ser Glu

305                 310                 315                 320

Ile Thr Val Thr Asp Glu Glu Val Ser Asn Pro Ser Cys Leu Asp Leu
                325                 330                 335

Val Arg Thr Pro Gln Met Arg Lys Cys Thr Leu Ile Leu Met Phe Ala
            340                 345                 350

Trp Phe Thr Ser Ala Val Val Tyr Gln Gly Leu Val Met Arg Leu Gly
        355                 360                 365

Leu Ile Gly Gly Asn Leu Tyr Ile Asp Phe Phe Ile Ser Gly Leu Val
    370                 375                 380

Glu Leu Pro Gly Ala Leu Leu Ile Leu Leu Thr Ile Glu Arg Leu Gly
385                 390                 395                 400

Arg Arg Leu Pro Phe Ala Ala Ser Asn Ile Val Ala Gly Val Ser Cys
                405                 410                 415

Leu Val Thr Ala Phe Leu Pro Glu Gly Ile Pro Trp Leu Arg Thr Thr
            420                 425                 430

Val Ala Thr Leu Gly Arg Leu Gly Ile Thr Met Ala Phe Glu Ile Val
        435                 440                 445

Tyr Leu Val Asn Ser Glu Leu Tyr Pro Thr Thr Leu Arg Asn Phe Gly
    450                 455                 460

Val Ser Leu Cys Ser Gly Leu Cys Asp Phe Gly Gly Ile Ile Ala Pro
465                 470                 475                 480

Phe Leu Leu Phe Arg Leu Ala Ala Ile Trp Leu Glu Leu Pro Leu Ile
                485                 490                 495

Ile Phe Gly Ile Leu Ala Ser Val Cys Gly Gly Leu Val Met Leu Leu
            500                 505                 510

Pro Glu Thr Lys Gly Ile Ala Leu Pro Glu Thr Val Glu Asp Val Glu
        515                 520                 525

Lys Leu Gly Ser Ser Gln Leu His Gln Cys Gly Arg Lys Lys Lys Thr
    530                 535                 540

Gln Val Ser Thr Ser Asp Val
545                 550


<210>  5
<211>  3502
<212>  DNA
<213>  Rattus norvegicus

<220>
<221>  CDS
<222>  (392)..(2047)
<223>

<400>  5
agcacagcgg cttggacccc tgcagcctaa gagcactgga ggctattcct gcaatcagca      60

gagtcctgag tctggcgagt ctgggtgatc ctagaactca gtgaactcca acttggagtg     120

accctttagg gcaggctata agtctgggtc cttggtgcac ggtgacactg ctgtgaaacc     180

accccaatcc gctctccggg tttacgccgg tcccgggcgc atacgggcag gaggccggga     240

gcttggagca ggaggagcag cccggggcgg gctgccaggg cgagcaagcg agcgaggcgg     300

gggcggcggg tgcagaactg cgggcggcgg cgcccgcggg tcactctcag gccgggcagc     360

agcgacaggg cgcagggtgg tggggcgcac c atg ccc acg ttc gac cag gcg     412
                                   Met Pro Thr Phe Asp Gln Ala
                                   1               5

ctg agg aag gcg ggc gag ttc ggg cgc ttc cag cgg cgc gtg ttc ctg     460
Leu Arg Lys Ala Gly Glu Phe Gly Arg Phe Gln Arg Arg Val Phe Leu
        10                  15                  20

ctg ctg tgc ctg acg ggt gtc acc ttc gcc ttc ctc ttc gtt ggt gtg     508
Leu Leu Cys Leu Thr Gly Val Thr Phe Ala Phe Leu Phe Val Gly Val
    25                  30                  35

gtc ttc ctg ggc agc cag ccc gac tac tat tgg tgt cgc ggg ccc cgc     556
Val Phe Leu Gly Ser Gln Pro Asp Tyr Tyr Trp Cys Arg Gly Pro Arg
40                  45                  50                  55

gcc acc gcg ctg gcc gag cgc tgc gcc tgg agc cct gag gag gag tgg     604
Ala Thr Ala Leu Ala Glu Arg Cys Ala Trp Ser Pro Glu Glu Glu Trp
                60                  65                  70

aac ctc acc acg ccg gag ctt cac gtc cca gct gag cgc cgt ggc caa     652
Asn Leu Thr Thr Pro Glu Leu His Val Pro Ala Glu Arg Arg Gly Gln
            75                  80                  85

ggc cac tgt cac cgc tac ctg ctg gaa gac acc aat acc agc tca gag     700
Gly His Cys His Arg Tyr Leu Leu Glu Asp Thr Asn Thr Ser Ser Glu
        90                  95                  100

ctc agc tgc gac cca ctc gct gcc ttc ccc aac cgc tcc gct cct ctg     748
Leu Ser Cys Asp Pro Leu Ala Ala Phe Pro Asn Arg Ser Ala Pro Leu
    105                 110                 115

gtg ccc tgc agc ggc gac tgg cgc tat gtg gag acc cac tcc acc atc     796
Val Pro Cys Ser Gly Asp Trp Arg Tyr Val Glu Thr His Ser Thr Ile
120                 125                 130                 135

gtc agc cag ttt gac ctt gtc tgc ggc aac gcc tgg atg ttg gac ctc     844
Val Ser Gln Phe Asp Leu Val Cys Gly Asn Ala Trp Met Leu Asp Leu
                140                 145                 150

acc caa gcc atc ttg aac ctt ggc ttc ctg gct ggg gct ttt acc ttg     892
Thr Gln Ala Ile Leu Asn Leu Gly Phe Leu Ala Gly Ala Phe Thr Leu
            155                 160                 165

ggc tat gca gcg gac aga tac ggc agg ctc atc gtt tac tta ata tcc     940
Gly Tyr Ala Ala Asp Arg Tyr Gly Arg Leu Ile Val Tyr Leu Ile Ser
        170                 175                 180

tgt ttc ggc gtt ggc atc acg gga gtt gtg gtg gcg ttt gca ccg aat     988
Cys Phe Gly Val Gly Ile Thr Gly Val Val Val Ala Phe Ala Pro Asn
    185                 190                 195

ttt tct gtg ttt gtg atc ttc cgc ttc ctg caa ggg gtg ttt gga aag     1036
Phe Ser Val Phe Val Ile Phe Arg Phe Leu Gln Gly Val Phe Gly Lys
200                 205                 210                 215

ggg gcc tgg atg act tgc ttt gtg att gtg aca gaa ata gtt ggt tcg 1084
Gly Ala Trp Met Thr Cys Phe Val Ile Val Thr Glu Ile Val Gly Ser
                220                 225                 230

aaa cag agg agg att gtg gga ata gtg atc cag atg ttc ttc acc ctc 1132
Lys Gln Arg Arg Ile Val Gly Ile Val Ile Gln Met Phe Phe Thr Leu
            235                 240                 245

gga atc atc att ctg ccc gga att gcc tac ttc acc ccc agc tgg cag 1180
Gly Ile Ile Ile Leu Pro Gly Ile Ala Tyr Phe Thr Pro Ser Trp Gln
        250                 255                 260

ggc atc cag cta gcc ata tcg ctg cct agc ttt ctc ttc ctc ctt tat 1228
Gly Ile Gln Leu Ala Ile Ser Leu Pro Ser Phe Leu Phe Leu Leu Tyr
    265                 270                 275

tac tgg gtg gtc cct gag tct ccc cgc tgg ctg atc acc cgg aag cag 1276
Tyr Trp Val Val Pro Glu Ser Pro Arg Trp Leu Ile Thr Arg Lys Gln
280                 285                 290                 295

gga gag aaa gcc ttg cag atc ctg agg cgt gtg gct aag tgc aat ggg 1324
Gly Glu Lys Ala Leu Gln Ile Leu Arg Arg Val Ala Lys Cys Asn Gly
                300                 305                 310

aaa cac ctc tcg tca aat tac tca gag atc aca gtt aca gat gaa gag 1372
Lys His Leu Ser Ser Asn Tyr Ser Glu Ile Thr Val Thr Asp Glu Glu
            315                 320                 325

gtc agt aac cca tcc tgt tta gac ctt gtg agg act ccc caa atg agg 1420
Val Ser Asn Pro Ser Cys Leu Asp Leu Val Arg Thr Pro Gln Met Arg
        330                 335                 340

aag tgc aca ctc att ctc atg ttt gct tgg ttc aca agt gcc gtg gtg 1468
Lys Cys Thr Leu Ile Leu Met Phe Ala Trp Phe Thr Ser Ala Val Val
    345                 350                 355

tat caa gga ctt gtc atg cgc ctg gga ctc atc gga ggc aac ctc tac 1516
Tyr Gln Gly Leu Val Met Arg Leu Gly Leu Ile Gly Gly Asn Leu Tyr
360                 365                 370                 375

atg gac ttc ttt atc tcg ggg ctc gtg gag ctg ccc gga gct ctc ttg 1564
Met Asp Phe Phe Ile Ser Gly Leu Val Glu Leu Pro Gly Ala Leu Leu
                380                 385                 390

atc ctt ctg acc att gag cgc ctt gga cga cgc ctt ccc ttt gcc gca 1612
Ile Leu Leu Thr Ile Glu Arg Leu Gly Arg Arg Leu Pro Phe Ala Ala
            395                 400                 405

agc aat ata gtg gca ggg gtg tcg tgt ctg gtc act gcc ttt tta cca 1660
Ser Asn Ile Val Ala Gly Val Ser Cys Leu Val Thr Ala Phe Leu Pro
        410                 415                 420

gaa ggg ata ccg tgg ctg agg acc aca gtc gct acc ttg gga aga cta 1708
Glu Gly Ile Pro Trp Leu Arg Thr Thr Val Ala Thr Leu Gly Arg Leu
    425                 430                 435

gga ata acc atg gcc ttt gaa att gtt tat ttg gta aat tca gag ttg 1756
Gly Ile Thr Met Ala Phe Glu Ile Val Tyr Leu Val Asn Ser Glu Leu
440                 445                 450                 455

tac cca acg aca tta cgg aac ttt ggg gtt tca ctc tgc tca ggc ctg 1804
Tyr Pro Thr Thr Leu Arg Asn Phe Gly Val Ser Leu Cys Ser Gly Leu

                460                 465                 470

tgt gac ttt ggg ggg atc ata gcc cca ttt ctg ctc ttt cga cta gca 1852
Cys Asp Phe Gly Gly Ile Ile Ala Pro Phe Leu Leu Phe Arg Leu Ala
            475                 480                 485

gcc ata tgg cta gaa ctg cct ctg atc atc ttt ggg atc ctg gca tct 1900
Ala Ile Trp Leu Glu Leu Pro Leu Ile Ile Phe Gly Ile Leu Ala Ser
        490                 495                 500

gtc tgt ggt ggc ctt gtg atg ctt ttg cct gaa act aag ggc atc gcc 1948
Val Cys Gly Gly Leu Val Met Leu Leu Pro Glu Thr Lys Gly Ile Ala
    505                 510                 515

ttg ccg gag acg gtg gaa gat gtg gaa aag ctt ggc agt tca cag ttg 1996
Leu Pro Glu Thr Val Glu Asp Val Glu Lys Leu Gly Ser Ser Gln Leu
520                 525                 530                 535

cac cag tgt ggc aga aaa aag aaa acc cag gtt tct act tct aat gtc 2044
His Gln Cys Gly Arg Lys Lys Lys Thr Gln Val Ser Thr Ser Asn Val
                540                 545                 550

tga ggcccctgag tctgcctgga agaccacaga agctgagctt ggaatatgcc 2097

ttgcagtgtg cttcacctcc tctaggtggc gccgttccaa ggaaccgtaa agattccaga 2157

gtattttata tggaataaga taaagattta tcatgctacc caagcttctg ggaacacata 2217

acctttgacc tctttagcaa agatccagta ggtacacaga ttctgcctag gttctctttc 2277

attgggacca atgtctctga cccactgggg aggagatatg agaaggtccc aaccagttct 2337

ccagttctca ggacagtccc atccaggaag caggatgaac atatcaaggc ctggcagtga 2397

acggaagtgt actcagctga tctgatcaag actgagcagt gaggagtgtt ctgaagagtc 2457

tgagaactgc tatttctgat tcgcttaagc tccgaataca tgccttcaga gagccgacat 2517

gcctggtgcc cctccccttc tgtccccaaa gccactgagg ttccaggccc caggttaggc 2577

acagttccct aaatgcaaca catccaagga cccagctaca ttattgtgaa atcacactgc 2637

agtccccaca ccccaatatt ttgtctcctc cttcagtgga ccgctataat cacagattct 2697

taattgagga agaaaaaaaa aacatggatt ttgatttccc gggccttaga atttgaaaaa 2757

taaaggggta attatgtcat atttgtgaat tccgtttcag tggtgcctgt ggctgagagt 2817

ccaaagaata gactttcttc atgccatctg ggcacttccg acttatcttg ggagaaaaac 2877

aaaacaccaa aaacaaatac aaatacagtt gttgtcactt catgataaaa gtcaattgta 2937

ttcatacttt aaaggattgg cgacccattt ataagggatg taggagatac taatgaggga 2997

ctgaattttt tttttattag atcaagactc ctcaagcaaa gactcaaatg taaacacggt 3057

attctacgtg gtgtgcagtt cttgataaat tttcggactc agtgtgtatg ttagactgaa 3117

agtttcttaa acttcaaact cagtgtggac aggcaaaagt agcctcaaag ggaaacctta 3177

ccgatgtgga agaaagaaat gcatatgtgg agaacataag acatgtgtgc caccccagag 3237

aagatgtgcg tgtgactgta aaacatcgtt tcaccctgac gcatgtgaga accgaatgtg 3297

agtgttcaga ctcttgacag ccttttgggt attcattgcc acacccactg tgcgtgcccg 3357

gcatctgtgg cctggttccc tgtgtgtttc atggctgtag cgcggctctt gtccactctg 3417

ctgcctttgg attgcatttc aagttggaag ttctgtttca taacaataaa caatttttaa 3477

actgttttta aaaaaaaaaa aaaaa 3502

<210> 6
<211> 551
<212> PRT
<213> Rattus norvegicus

<400> 6
Met Pro Thr Phe Asp Gln Ala Leu Arg Lys Ala Gly Glu Phe Gly Arg
1               5                   10                  15

Phe Gln Arg Arg Val Phe Leu Leu Leu Cys Leu Thr Gly Val Thr Phe
            20                  25                  30

Ala Phe Leu Phe Val Gly Val Val Phe Leu Gly Ser Gln Pro Asp Tyr
        35                  40                  45

Tyr Trp Cys Arg Gly Pro Arg Ala Thr Ala Leu Ala Glu Arg Cys Ala
    50                  55                  60

Trp Ser Pro Glu Glu Glu Trp Asn Leu Thr Thr Pro Glu Leu His Val
65                  70                  75                  80

Pro Ala Glu Arg Arg Gly Gln Gly His Cys His Arg Tyr Leu Leu Glu
                85                  90                  95

Asp Thr Asn Thr Ser Ser Glu Leu Ser Cys Asp Pro Leu Ala Ala Phe
            100                 105                 110

Pro Asn Arg Ser Ala Pro Leu Val Pro Cys Ser Gly Asp Trp Arg Tyr
        115                 120                 125

Val Glu Thr His Ser Thr Ile Val Ser Gln Phe Asp Leu Val Cys Gly
    130                 135                 140

Asn Ala Trp Met Leu Asp Leu Thr Gln Ala Ile Leu Asn Leu Gly Phe
145                 150                 155                 160

Leu Ala Gly Ala Phe Thr Leu Gly Tyr Ala Ala Asp Arg Tyr Gly Arg
                165                 170                 175

Leu Ile Val Tyr Leu Ile Ser Cys Phe Gly Val Gly Ile Thr Gly Val
            180                 185                 190

Val Val Ala Phe Ala Pro Asn Phe Ser Val Phe Val Ile Phe Arg Phe
        195                 200                 205

Leu Gln Gly Val Phe Gly Lys Gly Ala Trp Met Thr Cys Phe Val Ile
    210                 215                 220

Val Thr Glu Ile Val Gly Ser Lys Gln Arg Arg Ile Val Gly Ile Val
225                 230                 235                 240

Ile Gln Met Phe Phe Thr Leu Gly Ile Ile Ile Leu Pro Gly Ile Ala
                245                 250                 255

Tyr Phe Thr Pro Ser Trp Gln Gly Ile Gln Leu Ala Ile Ser Leu Pro
            260                 265                 270

Ser Phe Leu Phe Leu Leu Tyr Tyr Trp Val Val Pro Glu Ser Pro Arg
        275                 280                 285

Trp Leu Ile Thr Arg Lys Gln Gly Glu Lys Ala Leu Gln Ile Leu Arg
    290                 295                 300

Arg Val Ala Lys Cys Asn Gly Lys His Leu Ser Ser Asn Tyr Ser Glu
305                 310                 315                 320

Ile Thr Val Thr Asp Glu Glu Val Ser Asn Pro Ser Cys Leu Asp Leu
                325                 330                 335

Val Arg Thr Pro Gln Met Arg Lys Cys Thr Leu Ile Leu Met Phe Ala
            340                 345                 350

Trp Phe Thr Ser Ala Val Val Tyr Gln Gly Leu Val Met Arg Leu Gly
        355                 360                 365

Leu Ile Gly Gly Asn Leu Tyr Met Asp Phe Phe Ile Ser Gly Leu Val
    370                 375                 380

Glu Leu Pro Gly Ala Leu Leu Ile Leu Leu Thr Ile Glu Arg Leu Gly
385                 390                 395                 400

Arg Arg Leu Pro Phe Ala Ala Ser Asn Ile Val Ala Gly Val Ser Cys
                405                 410                 415

Leu Val Thr Ala Phe Leu Pro Glu Gly Ile Pro Trp Leu Arg Thr Thr
            420                 425                 430

Val Ala Thr Leu Gly Arg Leu Gly Ile Thr Met Ala Phe Glu Ile Val
        435                 440                 445

Tyr Leu Val Asn Ser Glu Leu Tyr Pro Thr Thr Leu Arg Asn Phe Gly
    450                 455                 460

Val Ser Leu Cys Ser Gly Leu Cys Asp Phe Gly Gly Ile Ile Ala Pro
465                 470                 475                 480

Phe Leu Leu Phe Arg Leu Ala Ala Ile Trp Leu Glu Leu Pro Leu Ile
                485                 490                 495

Ile Phe Gly Ile Leu Ala Ser Val Cys Gly Gly Leu Val Met Leu Leu
            500                 505                 510

Pro Glu Thr Lys Gly Ile Ala Leu Pro Glu Thr Val Glu Asp Val Glu
        515                 520                 525

Lys Leu Gly Ser Ser Gln Leu His Gln Cys Gly Arg Lys Lys Lys Thr
    530                 535                 540

Gln Val Ser Thr Ser Asn Val
545                 550

<210> 7
<211> 21
<212> DNA
<213> Artificial

<220>
<223>  an artificial sequence

<400>  7
tggtcgaacg tgggcatggt g                                                21

## Description

### Technical Field

The present invention relates to uses of the organic cation transporter OCT3 for therapy of mental disorders such as depression, anxiety neuroses, and drug dependencies.

### Background Art

Various pharmaceutical agents have been used in drug therapies; however, some of these agents become positive ions (cations) under biological conditions. In recent years, research involving transporters (transport proteins), which are located on the cell membrane and contribute to tissue translocation, absorption, renal excretion, and bile excretion of drugs by their active transport, has been significantly advanced (see Non-Patent Documents 1 and 2). At the same time, gene cloning and structural and functional analyses of organic cation transporters, which are involved in transporting ionic drugs, has also been significantly advanced.

Of these, the important transporters for transporting cationic drugs are the organic cation transporters (OCT) (see Non-Patent Document 3). Of these, OCT1 and OCT2 have been cloned in 1994 (see Non-Patent Document 4) and 1996 (see Non-Patent Document 5), respectively. Proximal renal tubular cells are known to play an important role in reducing the toxicity of organic cations by excreting organic cations into the urine (see Non-Patent Document 6). Both OCT1 and OCT2 are expressed in the kidneys, and therefore one of either OCT1 or OCT2 was predicted to be important; however, immunohistological study negated this possibility (see Non-Patent Documents 4 and 7), indicating the possibility that other OCTs are important.

OCT3 was cloned from rat placenta in 1998 as a transporter with high homology to OCT1 (see Non-Patent Document 8). The presence of OCT3 was clarified using gene sequences and pharmacological characteristics in the early 1990s, and it became clear that OCT3 was identical to the transporter known as uptake2, or extraneuronal monoamine transporter (EMT), which was cloned from human cardiac muscles at about the same time as OCT3 (see Non-Patent Document 9).

A series of studies further clarified that the OCT3 cation transport activity and tissue distribution differed from those of other OCTs. Specifically, immunohistological studies revealed that OCT3 was located not only in the placenta, but also in the kidneys, small intestine, lungs, heart, and brain, while OCT1 was specifically localized in the liver and kidneys and OCT2 was specifically localized in the kidneys (see Non-Patent Document 10). OCT3 was also revealed to be absent from nerve cells in the brain but present in nerve-supporting cells such as astrocytes (see Non-Patent Document 11) or at the blood-cerebrospinal fluid barrier (see Non-Patent Document 12). Further, OCT3 was clearly present in proximal renal tubular cells in kidneys, and is currently considered one of the most important transporters for the excretion of cationic drugs in the kidneys (see Non-Patent Document 13).

The substrate specificity of OCT3 is also vastly different from that of other OCTs. OCT3 is known to have the activity of transporting dopamine nerve toxin MPP+, noradrenaline, serotonin, dopamines, psychostimulants, anti-depressants, and such, while these are known to be not transported by other OCTs (see Non-Patent Document 10).

In addition to the above, the following findings have been reported to date:
1) In the brain*, in situ* hybridization shows OCT3 only in the area postrema, with low expression in other sites. Therefore, OCT3 is considered to have an important role in regulating nausea, appetite, and cardiovascular function (see Non-Patent Document 14).
2) OCT3 exists in glia cells and astrocytes, which are nerve-supporting cells, and has an important role in the regulation of monoamine concentration in the brain (see Non-Patent Document 15).
3) OCT3 and OCT1 are expressed in bronchial smooth muscle, and OCT3 is considered to be involved in acute bronchial contractions mediated by the suppression of norepinephrine intake due to steroid inhalation (see Non-Patent Documents 16 and 17Non-Patent Document).
4) Human OCT3 (also called EMT) has been confirmed to consist of haplotypes *1 to *12 in Caucasians (see Non-Patent Document 18).
5) OCT3 was suggested to be important for the uptake of 1-methyl-4-phenylpyridinium (MPP+) in cerebellar granule cells (see Non-Patent Document 19).
6) OCT3 expression is reduced by the administration of psychostimulants (see Non-Patent Document 20).
7) Expression of OCT3, which has the activity of transporting monoamines including serotonin, was increased compensatively in some sites of the brain in serotonin transporter deficient animals (see Non-Patent Document 21).
8) OCT3 and OCT2 were confirmed using kidney-derived HEK293 cells expressing OCT3/EMT to have the activity of transporting agmatine, which can be a parent compound for drugs to treat dependency (see Non-Patent Document 22).
9) Many kinds of transporters are present in the placenta and OCT3 is one of them (see Non-Patent Document 23).
10) OCT3/EMT is expressed in astrocytes, which are human nerve-supporting cells, and has an important role in the uptake of monoamines, drugs, and such (see Non-Patent Documents 24 and 25).
11) OCT3 is expressed and functional in gangliocytes in the upper uterine cervix (see Non-Patent Document 26).
12) OCT3/EMT is expressed at the brush border membrane side in small intestine-derived Caco-2 cells and has an important role in the uptake of substrates into cells (see Non-Patent Documents 27 and 28).
13) OCT1 and OCT3 are involved in the quantitative regulation of acetylcholine in the human placenta (see Non-Patent Document 29).
14) Madin-Darby canine kidney (MDCK) cells, a cultured cell line, are cells which express only OCT2, and expression of OCT1 and OCT3 is not detected (see Non-Patent Document 30).
15) OCT2 and OCT3 are expressed at the choroid plexus in brain, at which the blood-cerebrospinal fluid barrier is located, and OCT2 is important for transporting choline at this site (see Non-Patent Document 31).
16) The ability to transport MPP+, an OCT3/EMT substrate, is decreased by exposure to some P-glycoprotein ligands in kidney-derived HEK293 cells expressing OCT3/EMT (see Non-Patent Document 32).
17) The cation transport system is important for the uptake of agmatin in human glioma-derived SK-MG-1; however, there is a high possibility that it is not mediated by OCT3 (see Non-Patent Document 33).
18) Uptake of choline is observed in RBE4 cells, which are rat cerebral microvascular cells; however, OCT3 is not expressed and the involvement of an unidentified cation transport system is considered (see Non-Patent Document 34).
19) Uptake of MPP+, an OCT3/EMT substrate, in the heart is significantly decreased in OCT3-deficient mice constructed by gene modification; however, no change was observed in other tissues (see Non-Patent Document 35).
20) OCT3 (ORCT3 in mice) and MAO-A, a monoamine metabolic enzyme, are located at the same site in mouse placenta (see Non-Patent Document 36).
21) Rat OCT3 consists of 11 exons and 10 introns. Further, mouse OCT3 is 86% homologous with human OCT3. According to the immunohistological finding that OCT3 is localized in the proximal tubules, OCT3 is considered to have an important role in the excretion of cationic drugs in the kidney(see Non-Patent Document 13).
22) Cultured rat astrocytes express OCT3 (see Non-Patent Document 37).
23) The characteristics of MPP+ uptake differ completely between kidney derived Caki-1 cells, which contain OCT3, and primary cultured hepatocytes, which contain OCT1. Corticosterone suppresses the uptake of MPP+ in Caki-1 cells; however, the reduction in OCT3 function due to the increase of corticosterone in the blood during stress period is considered to have little influence on the change in monoamine uptake (see Non-Patent Document 38).
24) Sex hormones estradiol and progesterone significantly reduce noradrenaline uptake in cultured porcine tubular cells (see-Non-Patent Document 39).
25) OCT3 is identical to extraneuronal monoamine transporter "uptake 2" and is widely distributed throughout the brain, including the hippocampus, cerebral cortex, and cerebellum (see Non-Patent Document 40).
26) Uptake of adrenaline into cells involves OCT3/uptake2 and P-glycoprotein in primary cultured hepatocytes (see Non-Patent Document 41).
27) Reduced dopamine uptake in brain tissues due to oxidative stress involves a decrease in the uptake function of both the monoamine transport system (uptakel), located in neurons, and OCT3/uptake2 (see Non-Patent Document 42).
28) Voltage-sensitive cation transport protein OCT3 is isolated from rat placenta (see Non-Patent Document 43).
29) OCT3/EMT is located in human glia cells (see Non-Patent Document 44).
30) 1,1 '-diisopropyl-2,4'-cyanine (disprocynium24) is a potent inhibitor of OCT3/uptake2 and its intravenous administration strongly suppresses excretion of monoamine into urine (see Non-Patent Document 45).
31) Uptake2 has an important role in uptake of noradrenaline into rat cardiomyocytes (see Non-Patent Document 46).
32) The characteristics, tissue distribution, and such of OCT1 and OCT2, which are organic cation transporters like OCT3, have been summarized (see Non-Patent Document 49).

Further, human and mouse OCT3 genes have been compared, indicating the possibility of some relationship between an undiscovered OCT3 gene polymorphism and mental disorders; however, no concrete evidence has been presented (see Non-Patent Document 47).

Moreover, OCT3/EMT/uptake2 is a transporter discovered in rat cardiomyocytes and uses monoamines such as dopamines, serotonin, and noradrenaline as substrates. It is localized in cells other than neurons, and therefore OCT3/EMT/uptake2 is considered important in removing noradrenaline released on nerve stimulation at the periphery.

Anti-depressants have so far been reported as possible substrates for OCT3. Anti-depressants have superior inhibitory activity against the transport of MPP+, an OCT3 substrate; however, this is because part of the anti-depressant structure is similar to that of monoamine, and also because anti-depressants are powerful cationic drugs. Therefore, this drug tendency does not indicate a relationship between OCT3 and depression.

Another example of drugs discussed for a relationship between their drug efficacy and OCT3 are β blockers, which are antihypertensive drugs. This is because β blockers, which are antihypertensive drugs with a structure similar to monoamine, have the characteristic of being transported by OCT3, and OCT3 is well expressed in the heart. (Note that all of these ideas were dropped in the early to mid 1990s). These findings are said to have drawn attention because the substrate type and transporter expression coincided.

Kekuda *et al.,* who first cloned OCT3, reported that OCT3 expression is highest in the heart, followed by the lungs and kidneys, and is extremely low in the brain. Later, Wu *et al.,* who came from the same group, reported OCT3 expression in the brain, and several other groups reported OCT3 expression in nerve-supporting cells, such as glia cells; however, to date it has been considered futile to evaluate the function of OCT3 in the central nervous system, because OCT3 is expressed at low levels in the brain and is not expressed in nerves, and thus studies have not been conducted at this time.

Further, reports show no difference in the uptake of MPP+ in the brain of OCT3-deficient animals, and no abnormal behavior in OCT3-deficient animals. These results are also thought to be a reason for the lack of progress in research relating to the involvement of OCT3 in depression and anxiety
[Non-Patent Document 1] Otsuki, S. et al., Molecular mechanism of permeation and excretion of drugs in blood-brain barrier -central nervous support and protection system-, Nippon Yakurigaku Zasshi (Folia Pharmacologica Japonica). 2003; Vol. 122; p.55-64.
[Non-Patent Document 2] Endo, H., Molecular mechanism of drug transport. Nippon Yakurigaku Zasshi. 2000; Vol. 116; p114-124.
[Non-Patent Document 3] Koepsell, H. et al., Molecular pharmacology of organic cation transporters in kidney. J Membr Biol, 1999; Vol. 167; p.103-117.
[Non-Patent Document 4] Grundemann, D. et al., Drug excretion mediated by a new prototype of polyspecific transporter. Nature, 1994; Vol. 372; p.549-552.
[Non-Patent Document 5] Okuda, M. et al., cDNA cloning and functional expression of a novel rat kidney organic cation transporter, OCT2. Biochem. Biophys. Res. Commun. 1996; Vol. 224; p.500-507.
[Non-Patent Document 6] Pritchard, JB and Miller DS., Mechanisms mediating renal secretion of organic anions and cations. Physiol. Rev. 1993; Vol. 73; p.765-96.
[Non-Patent Document 7] Gorboulev, V. et al., Cloning and characterization of two human polyspecific organic cation transporters. DNA Cell Biol. 1997; Vol. 16; p.871-881.
[Non-Patent Document 8] Kekuda, R. et al., Cloning and functional characterization of a potential-sensitive, polyspecific organic cation transporter (OCT3) most abundantly expressed in placenta. J. Biol. Chem. 1998; Vol. 273; p.15971-15979.
[Non-Patent Document 9] Grundemann, D. et al., Molecular identification of the corticosterone-sensitive extraneuronal catecholamine transporter. Nat Neurosci. 1998; Vol. 1; p.349-351.
[Non-Patent Document 10] Wu, X. et al., Identity of the organic cation transporter OCT3 as the extraneuronal monoamine transporter (uptake2) and evidence for the expression of the transporter in the brain. J. Biol. Chem. 1998; Vol. 273; p.32776-32786.
[Non-Patent Document 11] Inazu, M. et al., Pharmacological characterization of dopamine transport in cultured rat astrocytes. Life Sci. 1999; Vol. 64; p.2239-2245.
[Non-Patent Document 12] Wakamatsu, K. et al., Mechanism of Brain barrier excretion of 1-methyl-4-phenylpyridinium (MPP+), The 123rd Annual Meeting of the Pharmaceutical Society of Japan, 2003; Abstract 4; p.64.
[Non-Patent Document 13] Wu, X. et al., Structure, function, and regional distribution of the organic cation transporter OCT3 in the kidney. Am J Physiol Renal Physiol. 2000; Sep. Vol. 279 (3); p.449-58.
[Non-Patent Document 14] Haag, C. et al., The localization of the extraneuronal monoamine transporter (EMT) in rat brain. J Neurochem. 2004; Jan. Vol. 88 (2); p.291-7.
[Non-Patent Document 15] Inazu. M. et al., The role of glial monoamine transporters in the central nervous system. Nihon Shinkei Seishin Yakurigaku Zasshi. 2003 Aug; Vol. 23(4); p.171-8.
[Non-Patent Document 16] Horvath, G. et al., Norepinephrine transport by the extraneuronal monoamine transporter in human bronchial arterial smooth muscle cells. Am J Physiol Lung Cell Mol Physiol. 2003 Oct; Vol. 285(4); p.829-37.
[Non-Patent Document 17] Horvath, G. et al., Steroid sensitivity of norepinephrine uptake by human bronchial arterial and rabbit aortic smooth muscle cells. Am J Respir Cell Mol Biol. 2001 Oct; Vol. 25(4); p.500-6.
[Non-Patent Document 18] Lazar, A. et al., Genetic variability of the extraneuronal monoamine transporter EMT (SLC22A3). J Hum Genet. 2003; Vol. 48(5); p.226-30.
[Non-Patent Document 19] Shang, T. et al., 1-Methyl-4-phenylpyridinium accumulates in cerebellar granule neurons via organic cation transporter 3. J. Neurochem. 2003 Apr; Vol. 85(2); p.358-67.
[Non-Patent Document 20] Kitaichi, K. et al., Increased plasma concentration and brain penetration of methamphetamine in behaviorally sensitized rats. Eur J Pharmacol., 2003 Mar 7; Vol. 464 (1); p.39-48.
[Non-Patent Document 21] Schmitt, A. et al., Organic cation transporter capable of transporting serotonin is up-regulated in serotonin transporter-deficient mice. J Neurosci Res. 2003 Mar 1; Vol. 71 (5); p.701-9.
[Non-Patent Document 22] Grundemann, D. et al., Agmatine is efficiently transported by non-neuronal monoamine transporters extraneuronal monoamine transporter (EMT) and organic cation transporter 2 (OCT2). J Pharmacol Exp Ther. 2003 Feb; Vol. 304 (2); p.810-7.
[Non-Patent Document 23] Leazer, TM., Klaassen, CD., The presence of xenobiotic transporters in rat placenta. Drug Metab Dispos. 2003 Feb; Vol. 31 (2); p.153-67.
[Non-Patent Document 24] Inazu, M. et al., Expression and functional characterization of the extraneuronal monoamine transporter in normal human astrocytes. J Neurochem. 2003 Jan; Vol. 84 (1); p.43-52.
[Non-Patent Document 25] Takeda, H. et al., Astroglial dopamine transport is mediated by norepinephrine transporter. Naunyn Schmiedebergs Arch Pharmacol. 2002 Dec; Vol. 366 (6); p.620-3.
[Non-Patent Document 26] Kristufek, D. et al., Organic cation transporter mRNA and function in the rat superior cervical ganglion. J Physiol. 2002 Aug 15; Vol. 543 (Pt 1); p.117-34.
[Non-Patent Document 27] Martel, F. et al., Uptake of (3) H-1-methyl-4-phenylpyridinium ((3) H-MPP (+)) by human intestinal Caco-2 cells is regulated by phosphorylation/dephosphorylation mechanisms. Biochem Pharmacol. 2002 Apr 15; Vol. 63 (8); p.1565-73.
[Non-Patent Document 28] Martel, F. et al., Apical uptake of organic cations by human intestinal Caco-2 cells: putative involvement of ASF transporters. Naunyn Schmiedebergs Arch Pharmacol. 2001 Jan; Vol. 363 (1); p.40-9.
[Non-Patent Document 29] Wessler, I. et al., Release of non-neuronal acetylcholine from the isolated human placenta is mediated by organic cation transporters. Br J Pharmacol. 2001 Nov; Vol. 134 (5); p.951-6.
[Non-Patent Document 30] Shu, Y. et al., Functional characteristics and steroid hormone-mediated regulation of an organic cation transporter in Madin-Darby canine kidney cells., J Pharmacol Exp Ther., 2001 Oct;Vol. 299 (1); p.392-8.
[Non-Patent Document 31] Sweet, DH. et al., Ventricular choline transport: a role for organic cation transporter 2 expressed in choroids plexus. J Biol Chem. 2001 Nov 9; Vol. 276 (45); p.41611-9.
[Non-Patent Document 32] Martel, F. et al., Effect of P-glycoprotein modulators on the human extraneuronal monoamine transporter. Eur J Pharmacol. 2001 Jun 22; Vol. 422 (1-3); p.31-7.
[Non-Patent Document 33] Molderings, GJ. et al., Agmatine and putrescine uptake in the human glioma cell line SK-MG-1. Naunyn Scmiedebergs Arch Pharmacol. 2001 Jun; Vol. 363 (6); p.671-9.
[Non-Patent Document 34] Friedrich, A. et al., Transport of choline and its relationship to the expression of the organic cation transporters in a rat brain microvessel endothelial cell line (RBE4). Biochim Biophys Acta. 2001 Jun 6; Vol. 1512 (2); p.299-307.
[Non-Patent Document 35] Zwart, R. et al., Impaired activity of the extraneuronal monoamine transporter system known as uptake-2 in Orct3/Slc22a3-deficient mice. Mol Cell Biol. 2001 Jul; Vol. 21 (13), p.4188-96.
[Non-Patent Document 36] Verhaagh, S. et al., The extraneuronal monoamine transporter Slc22a3/Orct3 co-localizes with the Maoa metabolizing enzyme in mouse placenta. Mech Dev. 2001 Jan; Vol. 100 (1); p.127-30.
[Non-Patent Document 37] Inazu, M. et al., Pharmacological characterization of dopamine transport in cultured rat astrocytes. Life Sci. 1999; Vol. 64 (24); p.2239-45.
[Non-Patent Document 38] Martel, F. et al., Comparison between uptake2 and rOCT1: effects of catecholamines, metanephrines and corticosterone. Naunyn Schmiedebergs Arch Pharmacol. 1999 Apr; Vol. 359 (4); p.303-9.
[Non-Patent Document 39] Dynarowicz, I. and Watkowski, T., The effect of oestradiol-17 beta and progesterone on uptake1, uptake2 and on release of noradrenaline in the uterine artery of ovariectomized pigs., Arch Vet Pol. 1993; Vol. 33 (3-4); p.249-58.
[Non-Patent Document 40] Wu, X. et al., Identity of the organic cation transporter OCT3 as the extraneuronal monoamine transporter (uptake2) and evidence for the expression of the transporter in the brain., J Biol Chem. 1998 Dec 4; Vol. 273 (49); p.32776-86.
[Non-Patent Document 41] Martel, F. et al., Uptake of [3H]-adrenaline by freshly isolated rat hepatocytes: putative involvement of P-glycoprotein. J Auton Pharmaco. 1998 Feb; Vol. 18 (1); p.57-64.
[Non-Patent Document 42] Page, G. et al., Possible relationship between changes in [3H] DA uptake and autoxidation in rat striatal slices. Exp Neurol. 1998 Jul; Vol. 152 (1); p.88-94.
[Non-Patent Document 43] Kekuda, R., et al., Cloning and functional characterization of a potential-sensitive, polyspecific organic cation transporter (OCT3) most abundantly expressed in placenta. J Biol Chem. 1998 Jun 26; Vol. 273 (26); p.15971-9.
[Non-Patent Document 44] Schomig, E., et al., The extraneuronal monoamine transporter exists in human central nervous system glia. Adv Pharmacol. 1998; Vol. 42; p.356-9
[Non-Patent Document 45] Graefe, KH. et al. 1, 1' -Diisopropyl-2,4'-cyanine (disprocynium24), a potent uptake2 blocker, inhibits the renal excretion of catecholamines. , Naunyn Schmiedebergs Arch Pharmacol. 1997 Jul; Vol. 356 (1); p.115-25.
[Non-Patent Document 46] Obst, OO. et al., Characterization of catecholamine uptake2 in isolated cardiac myocytes. Mol Cell Biochem. 1996 Oct-Nov; Vol. 163-164; p.181-3.
[Non-Patent Document 47] Wieland, A. et al. Analysis of the gene structure of the human (SLC22A3) and murine (Slc22a3) extraneuronal monoamine transporter. J Neural Transm. 2000; Vol. 107 (10); p.1149-57.
[Non-Patent Document 48] Lazar, A. et al., Genetic variability of the extraneuronal monoamine transporter EMT (SLC22A3). J Hum Genet. 2003; Vol. 48; p226-230.
[Non-Patent Document 49] J Pharmacol Exp Ther. 2004 Jan; Vol. 308 (1); p.2-9.

### Disclosure of the Invention

### Problems to Be Solved by the Invention

The present invention was conducted under the above situation. An objective of the present invention is to provide pharmaceutical agents for therapy of mental disorders such as depression, anxiety, or drug dependency, and to provide methods of screening for said agents, by revealing the relationship between mental disorders such as depression, anxiety, or drug dependency and OCT3.

### Means to Solve the Problems

As described above, there have been no reports to date of OCT3-deficient animals showing behavioral differences as compared to wild type. In view of the finding that the expression of OCT3, which has the activity of transporting monoamines including serotonin, was increased compensatively in some sites of brain in serotonin transporter deficient animals, the present inventors considered the following hypothesis. Specifically, they hypothesized that in the above OCT3-deficient animals, other transporters with monoamine transport activity were overexpressed during animal development, compensating for the function of OCT3. The present inventors further hypothesized that it would be possible to construct animals showing behavioral changes by suppressing OCT3 expression in the brain of mature animals. Thus, the present inventors conducted dedicated research to construct mice in which OCT3 expression is suppressed, resulting in behavioral change.

The present inventors constructed mice with suppressed OCT3 expression by directly administering antisense sequences against OCT3 into the brain. Although various sites can be considered as antisense target sites, in the present experiment sequences containing the start codon of the target gene OCT3 were used. Moreover, the present inventors focused on the finding that OCT3 was expressed in the blood-cerebrospinal fluid barrier, which is the contact point between the cerebral ventricle and the blood, and selected the cerebral ventricle as the site for administration of the above antisense sequences. Normally there are also methods that administer antisense sequences directly into target tissues when the target is located in the cerebral parenchyma, but generally, phosphorothioated antisense sequences, in which sulfurs are attached to phosphate groups, are often used to inhibit degradation of their nucleotide sequences. However, in consideration of the fact that the above phosphorothioated forms are toxic, often causing tissue death, and cause difficulties in experiments, the present inventors achieved antisense administration into the cerebral ventricle, which is inventive and innovative.

In order to examine the relationship between the mice constructed as described above and various mental disorders, the present inventors studied the effects on depression-like symptoms and anxiety symptoms. More specifically, they conducted forced swimming tests and observed exploratory behavior in the above mice. As a result, the present inventors revealed that the above mice showed no immobility during swimming and showed anti-depression effects. The present inventors also discovered that exploratory behavior was enhanced in the mice, and anti-anxiety effects were observed. Moreover, the present inventors confirmed that OCT3 expression was significantly decreased in these mice.

The results described above proved that substances (for example, antisense nucleic acids) which suppress OCT3 gene expression were actually effective against various mental disorders at the animal test level. That is, by the discoveries of the present invention, in which anti-depression effects or anti-anxiety effects were observed as behavioral changes in animals with suppressed OCT3 expression, substances suppressing OCT3 expression or function were shown to actually be effective as therapeutic agents for depression and anxiety disorders.

Further, the present inventors examined the combined effect of suppressing OCT3 gene expression and the use of anti-depressants. As a result, they newly discovered that the effect of anti-depressants is enhanced by the suppression of OCT3 expression. That is, compounds which regulate OCT3 expression were shown to be effective as concomitant drugs when used with anti-depressants.

Moreover, the present inventors discovered that the small molecule compounds which target OCT3 actually have the same effect as anti-depressants. That is, compounds which target OCT3 were shown to in fact have therapeutic effect against mental disorders such as depression.

Furthermore, the above results show that animals whose phenotype can be easily differentiated from that of a wild type can in fact be successfully constructed by suppressing the expression of a single gene (OCT3). As described above, the present inventors succeeded for the first time in constructing animals that could be easily differentiated from that of the wild type by suppressing expression of the OCT3 gene, thus completing the present invention. The animals of the present invention are extremely useful since they have phenotypes which relate to mental disorders such as depression and anxiety.

The above OCT3 gene knockout animals of the present invention are extremely useful, for example, for screening for therapeutic agents for mental disorders, or for identifying substances causing the mental disorders. Substances (compounds) obtained (identified) by the above methods can actually change (enhance or eliminate) the phenotypes of animals in the present invention, and can thus be said to be substances with therapeutic effects for mental disorders, or with very high probabilities of causing mental disorders.

Moreover, an increase in psychostimulant-induced spontaneous locomotor activity was observed in the above mice in which OCT3 expression was suppressed. That is, even a single administration of a psychostimulant showed the same behavior as the reverse tolerance phenomenon induced by the repeated psychostimulant administration. In the present invention, mice showing a psychostimulant-induced reverse tolerance phenomenon, that is, mice showing an increase in psychostimulant-induced spontaneous locomotor activity, were successfully constructed without repeated psychostimulant administration. The above animals are useful for analyses of the developmental mechanisms of drug-dependency. Further, the above animals can preferably be used for screening of therapeutic agents for therapy of psychostimulant dependency.

The present invention relates to OCT3 knockout animals with phenotypes associated with mental disorders such as depression, anxiety, and psychostimulant dependency. The present invention also relates to pharmaceutical agents for therapy of said mental disorders, and to methods of screening for said pharmaceutical agents. More specifically, the present invention provides:
[1] a therapeutic agent for a mental disorder comprising a substance suppressing the expression of an organic cation transporter OCT3 gene as an active ingredient;
[2] the therapeutic agent of [1], wherein the substance suppressing the expression of an organic cation transporter OCT3 protein is a compound selected from the group consisting of:
   (a) an antisense nucleic acid against a transcript of an OCT3 gene or a part thereof;
   (b) a nucleic acid with a ribozyme activity which specifically cleaves a transcript of an OCT3 gene; and
   (c) a nucleic acid with the action of inhibiting expression of an OCT3 gene by an RNAi effect;
[3] a therapeutic agent for a mental disorder comprising a substance suppressing a function of an organic cation transporter OCT3 protein as an active ingredient;
[4] the therapeutic agent of [3], wherein the substance suppressing a function of the organic cation transporter OCT3 protein is a compound of the following (a) or (b):
   (a) an antibody that binds to the organic cation transporter OCT3 protein; and
   (b) a small molecular compound that binds to the organic cation transporter OCT3 protein;
[5] the therapeutic agent of any one of [1] to [4], wherein the mental disorder is depression or an anxiety neurosis;
[6] a therapeutic agent for psychostimulant dependency, comprising a substance enhancing the expression or a function of an organic cation transporter OCT3 protein as an active ingredient;
[7] a gene knockout non-human animal, in which expression of an organic cation transporter OCT3 gene is artificially suppressed;
[8] the gene knockout non-human animal of [7], in which expression of the OCT3 gene is suppressed by the action of a nucleic acid of any one of:
   (a) an antisense nucleic acid against a transcript of an OCT3 gene or a part thereof;
   (b) a nucleic acid with a ribozyme activity which specifically cleaves an OCT3 gene transcript; and
   (c) a nucleic acid with the action of inhibiting expression of an OCT3 gene by an RNAi effect;
[9] the gene knockout non-human animal of [7], which shows a phenotype of any one of:
   (a) an anti-depression-like action;
   (b) an enhancement of psychostimulant-induced spontaneous locomotor activity; and
   (c) an anti-anxiety action;
[10] the gene knockout non-human animal of any one of [7] to [9], wherein the non-human animal is a rodent;
[11] a method of screening for an agent for therapy of a mental disorder, comprising the steps of:
   (a) contacting a test compound with an organic cation transporter OCT3 protein or its partial peptide;
   (b) measuring the binding activity of the protein or its partial peptide with the test compound; and
   (c) selecting a compound which binds to the organic cation transporter OCT3 protein or its partial peptide;
[12] a method of screening for an agent for therapy of a mental disorder, comprising the steps of:
   (a) contacting a test compound with a cell expressing an organic cation transporter OCT3 gene;
   (b) measuring the expression level of the organic cation transporter OCT3 gene; and
   (c) selecting a compound which reduces the expression level compared to that measured in the absence of the test compound;
[13] a method of screening for an agent for therapy of a mental disorder, comprising the steps of:
   (a) contacting a test compound with a cell comprising a DNA which comprises a structure in which a reporter gene is operably linked to a transcriptional control region of an organic cation transporter OCT3 gene;
   (b) measuring the expression level of the reporter gene; and
   (c) selecting a compound which reduces the expression level compared to that measured in the absence of the test compound;
[14] a method of screening for an agent for therapy of a mental disorder, comprising the steps of:
   (a) contacting a test compound with an organic cation transporter OCT3 protein or a cell or cell extract expressing the protein;
   (b) measuring an activity of the protein; and
   (c) selecting a compound which reduces the activity of the protein compared to that measured in the absence of the test compound;
[15] a method for identifying a compound causing a mental disorder, comprising the steps of:
   (a) administering a test compound to the gene knockout non-human animal of any one of [7] to [10];
   (b) observing a behavior dependent on a phenotype of the non-human animal; and
   (c) determining that a compound that abolishes the behavior dependent on the phenotype is a compound that causes a mental disorder;
[16] the method of any one of [11] to [15], wherein the mental disorder is depression or an anxiety neurosis;
[17] a method of screening for an agent for therapy of psychostimulant dependency, comprising the steps of:
   (a) contacting a test compound with a cell expressing an organic cation transporter OCT3 gene;
   (b) measuring the expression level of the organic cation transporter OCT3 gene; and
   (c) selecting a compound which increases the expression level compared to that measured in the absence of the test compound;
[18] a method of screening for an agent for therapy of psychostimulant dependency, comprising the steps of:
   (a) contacting a test compound with a cell comprising a DNA which comprises a structure in which a reporter gene is operably linked to a transcriptional control region of an organic cation transporter OCT3 gene;
   (b) measuring the expression level of the reporter gene; and
   (c) selecting a compound which increases the expression level compared to that measured in the absence of the test compound;
[19] a method of screening for an agent for therapy of psychostimulant dependency, comprising the steps of:
   (a) contacting a test compound with an organic cation transporter OCT3 protein or a cell or cell extract expressing the protein;
   (b) measuring an activity of the protein; and
   (c) selecting a compound which increases the activity of the protein compared to that measured in the absence of the test compound;
[20] a method of screening for an agent for therapy of psychostimulant dependency, comprising the steps of:
   (a) administering a test compound to the gene knockout non-human animal of any one of [7] to [10];
   (b) observing a psychostimulant-induced spontaneous locomotor activity that depends on a phenotype of the non-human animal; and
   (c) selecting a compound that abolishes the locomotor activity;
[21] a method for constructing the gene knockout non-human animal of [7], comprising a step of administering an antisense nucleic acid against a transcript of an OCT3 gene, or a part thereof, into the brain of a non-human animal;
[22] an anti-depressant action enhancing agent comprising a substance suppressing the expression of an organic cation transporter OCT3 gene as an active ingredient;
[23] the anti-depressant action enhancing agent of [22], wherein the substance suppressing the expression of the organic cation transporter OCT3 protein is a compound selected from the group consisting of:
   (a) an antisense nucleic acid against a transcript of an OCT3 gene or a part thereof;
   (b) a nucleic acid with a ribozyme activity which specifically cleaves a transcript of an OCT3 gene; and
   (c) a nucleic acid with the action of inhibiting the expression of an OCT gene by an RNAi effect;
[24] an anti-depressant action enhancing agent comprising a substance suppressing a function of an organic cation transporter OCT3 protein as an active ingredient;
[25] the anti-depressant action enhancing agent of [24], wherein the substance suppressing the function of the organic cation transporter OCT3 protein is a compound of the following (a) or (b):
   (a) an antibody which binds to the organic cation transporter OCT3 protein; and
   (b) a small molecular compound which binds to the organic cation transporter OCT3 protein; and
[26] a pharmaceutical composition with an anti-depressant effect comprising an anti-depressant and the anti-depressant action enhancing agent of any one of [22] to [25] as active ingredients.

Furthermore, the present invention provides:
[27] a method for treating and/or preventing a mental disorder, comprising the step of administering to an individual (for example, a patient or such) a substance suppressing the expression of an organic cation transporter OCT3 gene or a substance suppressing a function of an OCT3 protein;
[28] a method for treating and/or preventing psychostimulant dependency, comprising the step of administering to an individual (for example, a patient or such) a substance enhancing the expression of an organic cation transporter OCT3 gene or a substance enhancing a function of an OCT3 protein;
[29] use of a substance suppressing the expression of an organic cation transporter OCT3 gene or a substance suppressing a function of an OCT3 protein in the preparation of a therapeutic agent for a mental disorder; and
[30] use of a substance enhancing the expression of an organic cation transporter OCT3 gene or a substance enhancing a function of an OCT3 protein in the preparation of a therapeutic agent for psychostimulant dependency.

### Brief Description of the Drawings

Fig. 1 is graphs showing the effect in the depression model of continuous intracerebral infusion of the antisense against OCT3, showing the synergistic effect in the depression model of low dose antisense against OCT3 in combination with low dose imipramine, an anti-depressant. a: p<0.01 vs. a solvent group, b: p<0.01 vs. OCT3-ScrAS, c: p<0.01 vs. (OCT-AS 0 + IMI 0), d: p<0.01 vs. (OCT-AS 0 + IMI 4), e: p<0.01 vs. (OCT-AS 0.075 + IMI 0).
Fig. 2 is a graph showing the effect of continuous intracerebral infusion of normetanephrine, which is relatively selectively transported by OCT3, on the depression model. a: p<0.01 vs. a solvent group.
Fig. 3 is a graph showing a decrease in the expression of OCT3 protein in rats to which the antisense against OCT3 is continuously infused intracerebrally. a: p<0.01 vs. a solvent group, b: p< 0.01 vs. OCT3-ScrAS.
Fig. 4 is a graph showing the effect of continuous intracerebral infusion of the antisense against OCT3 on psychostimulant-induced spontaneous locomotor activity.
Fig. 5 is graphs showing the effect of continuous intracerebral infusion of antisense against OCT3 on anxiety activity. The graph on the left shows the count for rearing behavior as a result of the exploratory behavior. The graph on right shows the count for spontaneous locomotor activity.

### Best Mode for Carrying Out the Invention

The present invention revealed that substances which suppress the expression of the organic cation transporter OCT3 (sometimes simply referred to as "OCT3" in the present invention) have therapeutic effects against mental disorders such as depression and anxiety (neuroses). Therefore, the present invention relates to therapeutic agents for mental disorders, including substances which suppress the expression of the OCT3 gene or OCT3 protein or suppress the function (activity) of the protein (OCT3 protein) encoded by the OCT3 gene.

A preferred embodiment of the present invention provides therapeutic agents for mental disorders (agents and pharmaceutical compositions for treating mental disorders) which contain substances suppressing OCT3 gene expression as active ingredients.

The OCT3 of the present invention is known to be present in various organisms. OCT3 in the present invention includes OCT3 of various organisms. OCT3 in the present invention includes, for example, human OCT3, mouse OCT3, and rat OCT3. The nucleotide sequences of genes which code for these OCT3 are indicated in SEQ ID NO: 1 (human), SEQ ID NO: 3 (mouse), and SEQ ID NO: 5 (rat). Furthermore, the amino acid sequences of proteins which are encoded by these nucleotide sequences are indicated in SEQ ID NO: 2 (human), SEQ ID NO: 4 (mouse), and SEQ ID NO: 6 (rat). Other proteins with high homology to the sequences in the above sequence listing (normally 70% or more, preferably 80% or more, more preferably 90% or more, and most preferably 95% or more homology) and which have an OCT3 function (for example, a function of an organic cation transporter) are included as OCT3 in the present invention. The above proteins include, for example, proteins containing one or more amino acid additions, deletions, substitutions, or insertions in the amino acid sequence of the protein described in any one of SEQ ID NO: 2, 4, or 6. Moreover, the number of amino acid changes is normally 30 amino acids or less; preferably, ten amino acids or less; more preferably, five amino acids or less; and most preferably, three amino acids or less.

Examples of "mental disorders" on which the above therapeutic agents of the present invention are expected to have a therapeutic effect include depression, anxiety neuroses, manias, manic-depression, schizophrenia, and attention deficit hyperactive disorder (ADHD). The "mental disorders" in the present invention preferably include depression and anxiety neuroses.

Depression is a temporary mental condition or a chronic mental disorder generally characterized by feeling sadness, isolation, despair, and guilt, and covers disorders accompanied by symptoms such as psychomotor retardation, less frequent frustration, social withdrawal, and vegetative symptoms such as reduced appetite and insomnia. Furthermore, anxiety disorders generally refer to disorders with sudden anxiety attacks as a major symptom. Normally, they are accompanied by an elevated heart beat and pulse rate, breathing difficulty, dizziness, and shivering during an attack. So-called "panic disorders" are also included in the above anxiety neuroses.

Substances suppressing OCT3 gene expression in the present invention include, for example, substances which inhibit the transcription of OCT3 or inhibit translation from said transcript. Preferred embodiments of the above expression-suppressing substances include, for example, compounds (nucleic acids) selected from a group consisting of any of the following (a) to (c):
(a) antisense nucleic acids against OCT3 gene transcripts or parts thereof.
(b) nucleic acids with ribozyme activities which specifically cleave OCT3 gene transcripts.
(c) nucleic acid with the activity of inhibiting OCT3 gene expression by RNAi effect.

"Nucleic acids" in the present invention mean RNAs or DNAs. Moreover, chemically synthesized nucleic acid analogs such as so-called PNAs (peptide nucleic acids) are also included as nucleic acids in the present invention. In PNAs the pentose-phosphate backbone, which is the basic backbone structure of nucleic acids, is substituted with a polyamide backbone, built from glycine units. PNAs have a three-dimensional structure very similar to nucleic acids.

Methods well known to those skilled in the art for inhibiting the expression of a specific endogenous gene include those using antisense technology. Multiple factors contribute to the inhibition of target gene expression by antisense nucleic acids. These factors include, for example, inhibition of transcription initiation through triplex formation; inhibition of transcription through hybrid formation with a sequence at the site of a local open loop structure made by RNA polymerase; inhibition of transcription through hybrid formation with the RNA being synthesized; inhibition of splicing through hybrid formation with a sequence at an intron-exon junction; inhibition of splicing through hybrid formation with a sequence at a site of spliceosome formation; inhibition of transfer from the nucleus to the cytoplasm through hybrid formation with mRNAs; inhibition of splicing through hybrid formation with a sequence at the capping site or poly(A) site; inhibition of translation initiation through hybrid formation with a sequence at the site of binding of the translation initiation factor; inhibition of translation through hybrid formation with a sequence at the ribosome binding site near the initiation codon; inhibition of peptide chain elongation through hybrid formation with a sequence at the site of the translational region or polysome binding site of the mRNAs; and inhibition of gene expression through hybrid formation with a sequence at the site of interaction between proteins and nucleic acids. Thus, antisense nucleic acids inhibit target gene expression by inhibiting various processes, such as transcription, splicing and translation (Hirashima and Inoue, Shin Seikagaku Jikkenkoza 2 (New Lecture for Experimental Biochemistry 2), Kakusan IV (Nucleic Acid IV), Replication and Expression of Genes; Ed., Japanese Biochemical Society, Tokyo Kagaku Dozin Co., Ltd., pp. 319-347, 1993).

Antisense nucleic acids used in the present invention may inhibit the expression of an OCT3 gene through any one of the actions described above. In one embodiment, an antisense sequence is designed to be complementary to the 5'-untranslated region of an OCT3 gene mRNA. Thus such an antisense sequence is expected to effectively inhibit translation of that gene. Sequences complementary to the coding region or 3'-untranslated region can also be used for this purpose. Thus, nucleic acids comprising antisense sequences corresponding to sequences of translated as well as untranslated regions of OCT3 genes can be included as antisense nucleic acids used in the present invention. The antisense nucleic acids to be used are ligated downstream of an appropriate promoter, and are preferably ligated with a sequence comprising a transcription termination signal at the 3' end. The nucleic acids thus prepared can be used to transform desired animals by known methods.. The antisense nucleic acid sequences are preferably complementary to a target gene sequence or to a portion thereof; however perfect complementarity is not necessary as long as the antisense nucleic acid effectively inhibits target gene expression. The transcribed RNAs preferably have 90% or higher complementarity, and most preferably 95% or higher complementarity to the target gene transcript. The length of the antisense nucleic acids used to effectively inhibit target gene (OCT3) expression is preferably at least 15 nucleotides or longer and less than 25 nucleotides. However, the antisense nucleotides of the present invention are not limited to this length.

The antisenses in the present invention are not particularly limited; however, they can be constructed, for example, based on the nucleotide sequence from positions 388 to 408 of the rat OCT3 gene obtained from GenBank accession number NM_019230, or based on the nucleotide sequence from positions 377 to 397 of the mouse OCT3 gene obtained from GenBank accession number NM_011395. One example is RNAs which are complementary to the sequence 5'-tggtcgaacgtgggcatggtg-3' (SEQ ID NO: 7).

OCT3 gene expression can also be inhibited using ribozymes or ribozyme-encoding DNAs. Ribozymes refer to RNA molecules with catalytic activity. Ribozymes have a variety of activities, and studies focusing on ribozymes as RNA-cleaving enzymes have allowed the design of ribozymes that cleave RNAs in a site-specific fashion. Ribozymes such as group I intron-type ribozymes and M1 RNA, which are RNase P ribozymes, are 400 nucleotides or more in length. Others such as hammer-head and hairpin ribozymes have active sites comprising about 40 nucleotides (M. Koizumi and E. Otsuka, Tanpakushitsu Kakusan Koso (Protein, Nucleic acid, and Enzyme), 1990, 35, 2191).

For example, the autolytic domains of hammer-head ribozymes cleave the 3' side of C15 in the sequence G13U14C15. Base pairing between U14 and A9 plays an important role in this activity, and A15 or U15 can be cleaved instead of C15 (Koizumi, M. et al., FEBS Lett, 228: 228, 1988). Restriction enzyme-like RNA-cleaving ribozymes that recognize the target RNA sequences UC, UU or UA can be produced by designing the ribozymes such that a substrate binding site complements the RNA sequence near the target site (Koizumi, M. et al., FEBS Lett, 239: 285, 1988; M. Koizumi and E. Otsuka, Tanpakushitsu Kakusan Koso (Protein, Nucleic acid, and Enzyme), 35:2191, 1990; and Koizumi, M. et al., Nucl. Acids Res., 17: 7059, 1989).

Hairpin ribozymes can also be used for the purposes of the present invention. Such ribozymes are found, for example, in the minus strand of tobacco ring spot virus satellite RNA (Buzayan, J. M., Nature, 323: 349, 1986). Target specific RNA-cleaving ribozymes can also be produced from hairpin ribozymes (Kikuchi, Y. and Sasaki, N., Nucl. Acids Res., 19: 6751, 1991; Kikuchi, H., Kagaku to Seibutsu (Chemistry and Biology), 30: 112, 1992). Thus, in the present invention OCT3 gene expression can be inhibited by specifically digesting the OCT3 gene transcript using a ribozyme.

Inhibition of endogenous gene expression can be also performed by RNA interference (RNAi) using double stranded RNAs whose sequences are identical or similar to a target gene sequence. In the present invention, nucleic acids with inhibitory activity due to RNAi effect are also generally referred to as siRNAs. RNAi is a phenomenon in which destruction of a target gene mRNA can be induced and target gene expression can be suppressed by introducing cells and the like with double stranded RNAs consisting of sense RNAs homologous to an mRNA of the target gene and antisense RNAs consisting of their complementary sequences. Thus, RNAi can suppress the expression of target genes, and is therefore drawing attention as a simple method for knocking out genes, replacing conventional gene destruction methods using complicated and inefficient homologous recombination, and as a method applicable to gene therapy. RNAs used for RNAi do not necessarily have to be completely identical to an OCT3 gene or to a partial region of the gene; however, complete homology is preferred.

Preferable embodiments of the nucleic acids of (c), mentioned above, are double stranded RNAs with RNAi (RNA interference) effect (siRNAs) on an OCT3 gene. More specific examples comprise double stranded RNAs (siRNAs) comprising sense and antisense RNAs that correspond to a partial sequence of a nucleotide sequence of any one of SEQ ID NO: 1,3,or 5.

Although details of the mechanism of RNAi are still partially unclear, it is believed that an enzyme called DICER (a member of the RNase III nuclease family) contacts the double stranded RNAs, which are degraded into small fragments called small interfering RNAs, or siRNAs. The double stranded RNAs with RNAi effect of the present invention also comprise double stranded RNAs prior to such degradation by DICER. In other words, because even a long RNA, which by itself has no RNAi effect, is expected to be degraded within cells to form siRNAs that have an RNAi effect, the length of the double stranded RNAs of the present invention is not particularly limited.

For example, a long double stranded RNA corresponding to a full length, or almost full length region of an mRNA of an OCT3 gene of the present invention may be pre-degraded with DICER, and those degradation products can be used as drugs for therapy of mental disorders. Such degradation products are expected to comprise double stranded RNA molecules with RNAi effect (siRNAs). In such methods, it is not particularly necessary to select mRNA regions expected to have an RNAi effect. Thus, it is not particularly necessary to precisely define the region(s) on an mRNA of an OCT3 gene of the present invention that have an RNAi effect.

With respect to the above RNA molecules, molecules that have a closed structure on one end, for example, siRNAs comprising a hairpin structure (shRNAs), are also comprised in the present invention. Thus, single stranded RNA molecules that can form a double stranded RNA structure within the molecule are also comprised in the present invention.

Those skilled in the art can appropriately construct the above "double stranded RNAs capable of inhibiting through RNAi effect" of the present invention, based on the nucleotide sequences of the OCT3 genes of the present invention, which are targets for the double stranded RNAs. As an example, a double stranded RNA of the present invention may be constructed based on the nucleotide sequence of any of SEQ ID NO: 1, 3, or 5. Thus, based on the nucleotide sequence of any one of SEQ ID NO: 1, 3, or 5, it is within the range of standard practice for those skilled in the art to appropriately select an arbitrary continuous RNA region of an mRNA, which is a transcript of this nucleotide sequence, and construct a double stranded RNA corresponding to this region. Those skilled in the art can also use standard methods to appropriately select an siRNA sequence with a strong RNAi effect from the mRNA sequences which are the transcripts of this sequence. In addition, if one strand is determined (for example, the nucleotide sequence of any one of SEQ ID NO: 1, 3, or 5), those skilled in the art can easily know the nucleotide sequence of the other strand (the complementary strand). Those skilled in the art can appropriately generate siRNAs using commercially available nucleic acid synthesizers. Moreover, common custom synthesis services may be used to synthesize a desired RNA.

Furthermore, DNAs (vectors) that can express the above RNAs of the present invention are also comprised in a preferred embodiment of the compounds capable of inhibiting the expression of an OCT3 gene of the present invention. Examples of DNAs (vectors) that can express the above double stranded RNAs of the present invention are DNAs in which a DNA encoding one strand of the double stranded RNA and a DNA encoding the other strand of the double stranded RNA are linked to a promoter such that both can be expressed. The above DNAs of the present invention can be appropriately constructed by those skilled in the art using standard genetic engineering techniques. More specifically, the expression vectors of the present invention can be constructed by appropriately inserting DNAs encoding RNAs of the present invention into a variety of known expression vectors.

Furthermore, the expression-suppressing substances of the present invention include, for example, compounds which suppress OCT3 expression by binding to an OCT3 expression regulatory region (for example, a promoter region). The compounds can be obtained, for example, by screening methods using as an indicator the binding activity of compounds to DNA fragments of OCT3 promoters. Furthermore, those skilled in the art can appropriately determine whether or not the desired compounds can suppress the expression of OCT3 of the present invention using well known methods, including reporter assay methods.

Moreover, the present invention provides therapeutic agents for mental disorders which comprise substances suppressing the function of organic cation transporter OCT3 proteins as active ingredients. Suppressing the function of OCT3 protein as an organic cation transporter inhibits the transport of organic cations, including neurotransmitters, and thus elevates neurotransmitter function; hence it is considered effective in therapy of mental disorders. Moreover, compounds which suppress this function are considered effective as therapeutic agents for mental disorders.

The substances suppressing OCT3 protein function in the present invention include, for example, compounds of the following (a) or (b):
(a) antibodies which bind to an organic cation transporter OCT3 protein;
(b) small molecule compounds which bind to an organic cation transporter OCT3 protein.

The antibodies that bind to OCT3 proteins (anti-OCT3 antibodies) can be prepared by methods known to those skilled in the art. When the antibodies are polyclonal antibodies, they can be prepared, for example, using the following methods: Antiserum is obtained by immunizing small animals such as rabbits with a natural OCT3 protein, or a (recombinant) OCT3 protein expressed as a fusion protein with GST in microorganisms such as *E. coli,* or a partial peptide thereof. Antibodies are purified from the serum using, for example, ammonium sulfate precipitation, protein A columns, protein G columns, DEAE ion-exchange chromatography, affinity columns on to which an OCT3 protein or synthetic peptide thereof is immobilized, etc. Alternatively, monoclonal antibodies can be produced by, for example, immunizing small animals such as mice with an OCT3 protein or partial peptide thereof, removing the spleen, gently grinding the spleen to separate cells, fusing the cells with mouse myeloma cells using a reagent such as polyethylene glycol, and then screening the fused cells (hybridomas) to select clones that produce antibodies that bind to the OCT3 protein. These hybridomas are then transplanted into a mouse peritoneal cavity and ascites are collected from the same mouse. The monoclonal antibodies thus prepared can be purified using, for example, ammonium sulfate precipitation, protein A columns, protein G columns, DEAE ion-exchange chromatography, affinity columns on to which an OCT3 protein or synthetic peptide is immobilized, etc.

The forms of the antibodies of the present invention are not particularly limited so long as they can bind to OCT3 proteins of the present invention, and in addition to the aforementioned polyclonal antibodies and monoclonal antibodies, the antibodies include human antibodies, humanized antibodies obtained by genetic recombination, and antibody fragments and antibody modification products thereof.

The OCT3 proteins of the present invention, which are used as sensitizing antigens for acquiring antibodies, are not particularly limited in terms of the animal species from which they are derived; however, proteins of mammalian origin, such as proteins from mice or humans, are preferable, and proteins derived from human are particularly preferable. Those skilled in the art can appropriately obtain human-derived proteins using gene sequences or amino acid sequences disclosed in the present specification.

Proteins to be used as an immunization antigens in the present invention may be full-length proteins or partial peptides derived from those proteins. Such partial protein peptides include, for example, protein amino (N)-terminal fragments and carboxyl (C)-terminal fragments. As used herein, "antibodies" refer to antibodies which react with full-length proteins or fragments thereof.

In addition to obtaining the above hybridomas by immunizing non-human animals with antigens, hybridomas producing desired human antibodies with protein binding activity can also be prepared *in vitro* by sensitizing human lymphocytes, for example, human lymphocytes infected with EB virus, with a protein, cells expressing a protein, or a lysate of those cells, and fusing these sensitized lymphocytes with immortalized human myeloma cells, for example, U266 cells.

Antibodies against OCT3 proteins in the present invention inhibit the function of OCT3 proteins by binding to them, and the effect of treating or improving mental disorders can be expected, for example. Human and humanized antibodies are preferable when the obtained antibodies are to be administered to humans (antibody therapies) so as to reduce antigenicity.

Furthermore, as materials able to inhibit OCT3 protein function, the present invention includes low molecular weight materials (small molecule compounds) which bind to OCT3 proteins. The low molecular weight materials which are able to bind to OCT3 protein in the present invention may be natural or artificial compounds. Generally, they can be synthesized or obtained by methods well known to those skilled in the art. Moreover, the compounds in the present invention can be obtained by screening methods described below.

Furthermore, compounds which are substrates for OCT3 proteins are considered to have the potential to competitively inhibit the transporter activity of OCT3. For example, β blockers (therapeutic drugs for heart failure or such) such as propranorol are known to be transported by OCT3. Beta blockers cannot pass through the blood-brain barrier, however, β blocker analogs that can be transported to the central nervous system and that competitively inhibit the transport activity of OCT3 are expected to be able to inhibit the function of the above OCT3 protein in the brain. That is, such inhibitors are also included as the above small molecule compounds of the present invention.

The small molecule compounds of the above (b) which bind to OCT3 proteins include, for example, compounds with a high affinity for OCT3. Examples of the compounds include normetanephrine, which is an inactive metabolite of noradrenaline. Normetanephrine is a preferred example of the above small molecule compounds since it has been actually confirmed to have anti-depression activity, as described below in the Examples.

Furthermore, other than normetanephrine, examples of small molecule compounds with affinity for OCT3 include 3-methoxyisoprenaline, 3-O-methyl isoprenaline, carteolol, (-) isoprenaline, (-) adrenaline, 1-methyl-4-phenylpyridinium (MPP+), (2-chloroethyl)-3-sarcosinamide-1-nitrosourea (SarCNU), 1,1'-diisopropyl-2,4'-cyanine (disprocynium 24), decynium 22, cyanine 863, corticosterone, estradiol, disopyramide, lidocaine, and procainamide.

Moreover, the above compounds include compounds with various pharmacological effects mediated by other targets, that do not target OCT3 alone. For example, 3-methoxyisoprenaline, 3-O-methyl isoprenaline, carteolol, (-) isoprenaline, and (-) adrenaline have anti-hypertension effects; SarCNU has anti-tumor effects; disopyramide, lidocaine, and procainamide have anti-arrhythmic effects; and corticosterone and estradiol have steroid hormone-like effects. Furthermore, 1-methyl-4-phenylpyridinium (MPP+) is known as a dopamine nerve toxin. Moreover, disprocynium 24, decynium 22, and cyanine 863 are potent OCT3 inhibitors; however, at the same time they show potent inhibitory activity towards other OCT subtypes.

Therefore, when the above compounds, excluding normetanephrine, are used as pharmaceutical agents of the present invention, for example, it is preferred to remove affinity to targets other than OCT3 by derivatizing the above compounds. Thus, derivatives of the various compounds described above are also useful as the small molecule compounds of the present invention.

A preferred embodiment of the present invention provides therapeutic agents for mental disorders which contain compounds with affinity for OCT3 that are selected from the group consisting of normetanephrine, 3-methoxyisoprenaline, 3-O-methyl isoprenaline, carteolol, (-) isoprenaline, (-) adrenaline, 1-methyl-4-phenylpyridinium (MPP+), (2-chloroethyl)-3-sarcosinamide-1-nitrosourea (SarCNU), 1,1'-diisopropyl-2,4'-cyanine (disprocynium 24), decynium 22, cyanine 863, corticosterone, estradiol, disopyramide, lidocaine, and procainamide, or derivatives of these compounds as active ingredients.

Furthermore, materials able to inhibit the function of OCT3 proteins in the present invention include OCT3 protein mutants with dominant negative characteristics against OCT3 proteins. "OCT3 protein mutants with dominant negative characteristics against OCT3 protein" are proteins with the function of diminishing or decreasing endogenous wild type protein activity by expressing the gene encoding that protein.

Furthermore, the function-suppressing substances of the present invention can be properly obtained by screening methods that use the cation transporter activity of OCT3 in the present invention as an indicator.

Moreover, the present invention provides therapeutic agents for psychostimulant dependency containing materials which enhance the expression or function (activation) of an organic cationic transporter OCT3 protein as active ingredients. The above "enhancement in protein expression" includes enhancement of transcription from the gene, enhancement of translation from the transcript, and such.

Generally, psychostimulants are central nervous system stimulants used to remain awake and to eliminate fatigue, and they usually refer to methamphetamines or to synthetic drugs similar to methamphetamines. Psychostimulants in the present invention also include compounds other than the above methamphetamines, and psychostimulant analogues and such are also included. For example, psychostimulants other than methamphetamine include amphetamines and MDMA. Moreover, psychostimulant analogues include, for example, methylphenidate (brand name: Ritalin).

Amphetamines and methamphetamines have very similar chemical structures and cause the same pharmacological effects. Most of the psychostimulants whose abuse has become a problem in Japan are methamphetamines, which are usually illegally sold and abused in its hydrochloride form. Methamphetamines, to which a methyl group is attached, have higher pharmacological potency.

The use of psychostimulants causes an increase in heart rate, breathing, and blood pressure, pupil dilation, and a decrease in appetite. Dependency develops with continuous use of psychostimulants and symptoms of dependency include sweating, headache, blurred vision, dizziness, insomnia, anxiety, and such, as well as increased sensitivity (reversed tolerance) to psychostimulants. The reversed tolerance phenomenon is sustained for long periods and readily reappears in spite of long-term withdrawal after continuous use of psychostimulants. It is not treatable with existing therapeutic agents for mental disorders, and therefore is considered to be the result of an irreversible change in neuronal function. An increase in psychostimulant-induced spontaneous locomotor activity is observed upon the repeated administration of psycho stimulants in experimental animals, and this phenomenon cannot be treated with the existing therapeutic agents for mental disorders. Therefore, substances which can increase spontaneous locomotor activity as a replacement for psychostimulants are effective as agents for treating psychostimulant dependencies. In the present invention, when the expression of OCT3 protein was suppressed in mice, the same behavior as a reversed tolerance phenomenon, which is caused by the repeated administration of psychostimulants, was observed. That is, agents for enhancing the expression or the function (activity) of OCT3 protein are effective as therapeutic agents for psychostimulant dependency.

Those skilled in the art could obtain the above function-enhancing agents of the present invention using the reporter assays described above or by methods which use the transporter activity of OCT3 as an indicator.

Moreover, the substances suppressing OCT3 gene expression and the substances suppressing OCT3 protein function of the present invention themselves have a therapeutic effect on mental disorders such as depression and anxiety neuroses; however, for example, they also have the activity of enhancing the effect of anti-depressants when used together with known anti-depressants.

Therefore, the present invention provides anti-depressant activity-enhancing agents (anti-depressant concomitant agents) which comprise substances suppressing the OCT3 gene expression or substances suppressing the OCT3 protein function as active ingredients. Moreover, the present invention relates to pharmaceutical compositions which comprise anti-depressants and the anti-depressant activity-enhancing agents of the present invention as active ingredients.

Examples of anti-depressants whose action (effect) is enhanced by combined use with the anti-depressant action-enhancing agents of the present invention are imipramine, classical anti-depressants including tricyclic anti-depressants whose structure is similar to imipramine, selective serotonin re-uptake inhibitors (SSRI), and serotonin and noradrenaline re-uptake inhibitors (SNRI).

Furthermore, the present invention provides OCT3 knockout non-human animals (herein sometimes referred to as "knockout non-human animals" or simply "animals"), characterized by the artificial suppression of expression of an organic cation transporter OCT3 gene.

The gene knockout non-human animals of the present invention can be used to screen drugs for therapy of mental disorders such as depression and anxiety neuroses. Moreover, they are very useful as disease animal models for analyzing the mechanisms of the above diseases.

The knockout animals of the present invention include so-called "knockdown animals" in which expression of genes is suppressed by the action of antisense RNAs or siRNAs.

"OCT3 gene expression is artificially suppressed" as used herein refers to, for example, (1) conditions where the expression of OCT3 gene is suppressed by mutations such as an insertion, deletion, and substitution in one or both gene pairs of the OCT3 gene, (2) conditions where the expression of the gene is suppressed by the action of the nucleic acids (for example, antisense RNAs or siRNAs) of the present invention.

The term "suppression" as used herein includes complete suppression of OCT gene expression and significantly reduced amounts of OCT3 expression in the animals of the present invention compared with wild type animals.

The above (1) includes suppression of the expression of only one gene of the gene pair of an OCT3 gene. The sites of gene mutations are not limited in the present invention, as long as a site is such that expression of the gene can be suppressed. For example, exon sites and promoter regions are included.

Those skilled in the art can generate the knockout animals of the present invention using commonly known genetic engineering techniques. For example, a knockout mouse can be generated as follows: first, a DNA comprising an exon portion of an OCT3 gene of the present invention is isolated from mice and an appropriate marker gene is inserted to this DNA fragment to construct a targeting vector. The targeting vector is introduced into a mouse ES cell line by electroporation or such, and cell lines in which homologous recombination has taken place are selected. The marker gene to be inserted is preferably an antibiotic resistance gene, such as the neomycin resistance gene. When an antibiotic resistance gene is inserted, cell lines in which homologous recombination has taken place can be selected by simply culturing cells in a medium comprising the antibiotic. Furthermore, more efficient selection is possible when a thymidine kinase gene or such is linked to the targeting vector. By doing this, cell lines in which non-homologous recombination had taken place can be eliminated. Furthermore, homologous recombinants may be examined by PCR and Southern blotting to efficiently obtain cells lines in which one of a gene pair of a gene of the present invention is inactivated.

When selecting cell lines in which homologous recombination has taken place, since there is a risk of unknown gene destruction in sites other than the site of homologous recombination due to gene insertion, it is preferable to use multiple clones to generate chimera. Chimeric mice can be obtained by injecting the obtained ES cell lines into mouse blastoderms. By crossing these chimeric mice, mice in which one of a gene pair of an OCT3 gene of the present invention is inactivated can be obtained. Further, by crossing these mice, mice in which both of the gene pair of a gene of the present invention are inactivated can be obtained. Similar procedures may be employed to genetically modify animals other than mice for which ES cells are established.

The above knockout animals of the present invention are preferably knockout (knockdown) animals characterized by suppressed OCT3 gene expression due to the introduction of the above nucleic acids of the present invention into non-human animals.

The above knockdown animals can be constructed by introducing non-human animals with a vector structured such that the nucleic acids (antisense RNAs, siRNAs, or such) of the present invention can be expressed.

Moreover, methods for constructing the knockout non-human animals of the present invention constructed as above are also included in the present invention. A preferred embodiment of the present construction methods is methods for constructing knockout non-human animals that include the step of administering the nucleic acids of the present invention into the brains of animals of the present invention. More specifically, for example, the methods include the step of administering antisense nucleic acids against an OCT gene transcript or part thereof into the brains, and preferably into the cerebral ventricles, of the animals of the present invention. Administration can be performed, for example, by the methods described in

### Examples.

The above antisense nucleic acids of the present invention are not particularly limited; however, for example, they are preferably designed as follows: (1) they are antisense sequences containing a start codon of the target gene, OCT3; (2) the appropriate length of an antisense sequence is an 18-25mer; (3) the length upstream from the start codon is preferably not too long since there is a possibility that the transcriptional control region will be covered; (4) designs which might cause the antisenses to form monomers (single strands bind to themselves) or dimers (two antisense strands bind to each other) are avoided.

The types of knockout animals are not limited in the present invention, as long as they are non-human animals; however, they are usually mammals, and preferably primates. More specifically, the animals in the present invention are preferably rodents such as mice, rats, and hamsters (rat species), or monkeys, and more preferably mice or monkeys.

In a preferred embodiment, the knockout non-human animals of the present invention are not limited; however, OCT3 gene expression in the animals is suppressed by the action of any of the nucleic acids of (a) to (c).
(a) antisense nucleic acids against an OCT3 gene transcript or part thereof.
(b) nucleic acids with ribozyme activity which specifically cleave an OCT3 gene transcript.
(c) nucleic acids with the activity of inhibiting OCT3 gene expression using RNAi effect.

OCT3 gene knockout (knockdown) non-human animals in the present invention are animals characterized by showing phenotypes related to mental disorders such as depression, anxiety neuroses, and psychostimulant dependency. More specifically, the animals of the present invention are gene knockout non-human animals characterized, for example, by showing at least one of the following phenotypes (a) to (c):
(a) anti-depression-like actions
(b) increased psychostimulant-induced spontaneous locomotor activity
(c) anti-anxiety actions

When experimental animals are made to swim in containers that prevented them from touching the bottom and from crawling out, and then made to swim again in the same container (forced swimming tests), the animals fall into despair state and show immobility for most of the experiment time. This condition is thought to be "depression". Animals in which this immobility ("depressed condition") is improved or completely diminished are examples of animals of the present invention showing a phenotype of the above (a): "anti-depression-like actions".

Dependency develops upon repeated use of psychostimulants and such symptoms include increased sensitivity (reversed tolerance) to psychostimulants. An increase in psychostimulant-induced spontaneous locomotor activity is also observed upon the repeated administration of psychostimulants to experimental animals. Animals with this condition are an example of animals of the present invention showing the above phenotype (b): "increased psychostimulant-induced spontaneous locomotor activity".

Moreover, animals initially show exploratory behaviors (ambulation and rearing behaviors) when exposed to new, open spaces; however, such behaviors are reduced with time. Animals in which such behaviors are diminished and exploratory behaviors are maintained are examples of animals of the present invention showing the phenotype of the above (c): "anti-anxiety actions".

The above animals of the present invention are extremely useful for screening of therapeutic agents for mental disorders, for identification of causative agents of the mental disorders, and for analyzing the mechanisms of the development of chemical dependencies.

Moreover, the present invention provides methods of screening for agents for the therapy of mental disorders (for example, depression and anxiety neuroses) and psychostimulant dependency, and provides methods for identifying compounds causing the mental disorders. The above "agents for therapy" also include agents concomitant with the therapeutic drugs (therapeutic drug action-enhancing agents). Therefore, one of the embodiments of the present invention relates to methods of screening for concomitant drugs used for the therapy of mental diseases (for example, depression and anxiety neuroses) or psychostimulant dependencies (for example, therapeutic drug action-enhancing agents).

A preferred embodiment of the screening methods of the present invention is a method in which binding to the organic cation transporter OCT3 protein or to its partial peptides is used as an indicator. Generally, compounds which bind to OCT3 protein or to its partial peptides are expected to have the effect of inhibiting OCT3 protein function.

In more detail, the above methods of the present invention comprise the following steps (a) to (c):
(a) contacting an organic cation transporter OCT3 protein or its partial peptide with a test compound;
(b) measuring the binding activity of the protein or its partial peptide with the test compound; and
(c) selecting a compound which binds to the organic cation transporter OCT3 protein or its partial peptide.

In the above methods of the present invention, the test compounds are first contacted with an OCT3 protein or its partial peptides. The OCT3 protein or its partial peptides can be, for example, purified forms, forms expressed intracellularly or extracellularly, or forms bound to an affinity column, depending on the indicator for detecting the binding of the test compounds with the OCT3 protein or its partial peptides. The test compounds used in these methods can be properly labeled as necessary. The labels include radio labels, fluorescent labels, and such.

Next in the present invention, the binding activity of the test compounds with the OCT3 protein or its partial peptides is measured. The binding of a test compound to an OCT3 protein or its partial peptide can be detected, for example, using a label attached to the test compound which is bound to the OCT3 protein or its partial peptide. Alternatively, changes in OCT3 protein activity can be detected as an indicator, where the changes are induced when test compounds bind to the OCT3 protein or its partial peptides, expressed intracellularly or extracellularly.

The test compounds which bind to the OCT3 protein or its partial peptides are then selected in the present invention.

The compounds selected (obtained) by the present methods are expected to have an inhibitory effect against OCT3 protein. For example, they are expected to be drugs for the therapy of mental disorders (for example, therapeutic drugs or therapeutic drug action-enhancing agents).

In another embodiment of the screening methods of the present invention, the expression level of an OCT3 gene is used as a signal. Compounds that decrease OCT3 gene expression level are expected to be agents for the therapy of mental disorders. On the other hand, compounds that increase OCT3 gene expression level are expected to be agents for the therapy of psychostimulant dependencies.

The above methods of the present invention are methods of screening for agents for the therapy of mental disorders. For example, the methods comprise the following steps (a) to (c):
(a) contacting cells expressing an organic cation transporter OCT3 gene with a test compound;
(b) measuring the expression level of said organic cation transporter OCT3 gene; and
(c) selecting a compound which decreases said expression level compared with that measured in the absence of the test compound.

Furthermore, the above methods of the present invention are methods of screening for agents for the therapy of psychostimulant dependencies. For example, the methods comprise the following steps (a) to (c):
(a) contacting cells expressing an organic cation transporter OCT3 gene with a test compound;
(b) measuring the expression level of said organic cation transporter OCT3 gene; and
(c) selecting a compound which increases said expression level compared with that measured in the absence of the test compound.

In the present invention, the test compounds are first contacted with cells expressing an OCT3 gene. The origin of the "cells" used includes cells derived from humans, mice, rats, and such, but is not limited thereto. Cells expressing an endogenous OCT3 gene or cells expressing an exogenous OCT3 gene which has been introduced into the cells can be used as the "cells expressing an OCT3 gene". The cells expressing an exogenous OCT3 gene can generally be constructed by introducing host cells with an expression vector containing an OCT3 gene. Such expression vectors can be constructed by general genetic engineering techniques.

The test compounds used in the present methods are not particularly limited and include, for example, single compounds such as natural compounds, organic compounds, inorganic compounds, proteins, and peptides, as well as chemical libraries, the expressed products from gene libraries, cell extracts, supernatants from cell cultures, bacterial fermentation products, marine organism extracts, and plant extracts.

Typically, but without limitation, a test compound is contacted with cells expressing an OCT3 gene by adding the test compound to a culture medium of the cells expressing the OCT3 gene. When the test compound is a protein, the contact can be achieved by introducing into the cells a DNA vector that allows protein expression.

The next step of these methods comprises determining the expression level of the OCT3 gene. Herein, the phrase "gene expression" refers to both transcription and translation. The gene expression level can be determined using methods known to those skilled in the art. For example, mRNAs can be extracted from cells expressing an OCT3 gene according to conventional methods, and by using this mRNA as a template, the transcriptional level of the gene can be determined using Northern hybridization or RT-PCR. Alternatively, the translational level of the gene can be determined by collecting protein fractions from the cells expressing the OCT3 family gene, and then expression of the OCT3 protein can be detected using an electrophoresis method such as sodium dodecyl sulfate-polyacrylamide gel electrophoresis (SDS-PAGE). Furthermore, the translational level of the gene can be determined by detecting the expression of the OCT3 protein by Western blotting analysis using antibodies against the protein. The types of antibodies used for OCT3 protein detection are not limited, as long as the protein can be detected. Such antibodies include, for example, both monoclonal and polyclonal antibodies.

Next in the present methods, compounds that increase or decrease the expression level compared with that measured when the test compounds are not contacted (controls) are selected. The compounds that cause a decrease are agents for therapy of mental disorders, while the compounds that cause an increase are agents for therapy of psychostimulant dependencies.

Another embodiment of the screening methods of the present invention are methods for identifying compounds which decrease or increase the expression level of an organic cation transporter OCT3 gene of the present invention by using the expression of a reporter gene as an indicator.

The above methods of the present invention are methods of screening for agents for therapy of mental disorders comprising, for example, the following steps (a) to (c);
(a) contacting a test compound with cells comprising a DNA having a structure such that a reporter gene is operably linked to a transcriptional control region of an organic cation transporter OCT3 gene;
(b) measuring the expression level of said reporter gene; and
(c) selecting a compound which decreases said expression level compared with that measured in the absence of the test compound.

Furthermore, the above methods of the present invention are methods of screening for agents for therapy of psychostimulant dependencies, comprising, for example, the following steps (a) to (c):
(a) contacting a test compound with cells comprising a DNA having a structure such that a reporter gene is operably linked to a transcriptional control region of an organic cation transporter OCT3 gene;
(b) measuring the expression level of said reporter gene; and
(c) selecting a compound which increases said expression level compared with that measured in the absence of the test compounds.

In these methods, test compounds are first contacted with cells (or extracts of these cells) that comprise a DNA with a structure in which a reporter gene is operably linked to a transcriptional regulatory region of an OCT3 gene. As used herein, the phrase "operably linked" means that the transcriptional regulatory region of an OCT3 gene is linked to a reporter gene in such a way as to induce reporter gene expression when a transcriptional factor binds to the transcriptional regulatory region of the OCT3 gene. Thus, even when the reporter gene is connected with another gene and thus forms a fusion protein with that gene product, such a connection can be expressed by the phrase "operably linked", as long as the expression of the fusion protein is induced when the transcriptional factor binds to the transcriptional regulatory region of the OCT3 gene. Using known methods and based on cDNA nucleotide sequences of OCT3 genes, those skilled in the art can obtain from the genome the transcriptional regulatory region of OCT3 genes.

The types of reporter genes used in these methods are not limited, as long as its expression can be detected. Such reporter genes include, for example, the CAT gene, lacZ gene, luciferase gene, and GFP gene. The "cells that comprise a DNA structured such that a reporter gene is operably linked to a transcriptional regulatory region of an OCT3 gene" include, for example, cells introduced with a vector inserted with such a structure. Those skilled in the art can prepare such vectors using routine genetic engineering techniques. Such a vector can be introduced into cells using conventional methods, for example, using calcium phosphate precipitation, electroporation, the lipofectamine method, microinjection, and so on. The "cells that comprise a DNA structured such that a reporter gene is operably linked to a transcriptional regulatory region of an OCT3 gene" also include cells with that structure has been inserted into their chromosome. The DNA structures can be inserted into chromosomes using methods routinely used by those skilled in the art, for example, gene transfer methods using homologous recombination.

Extracts of these "cells that comprise a DNA structured such that a reporter gene is operably linked to a transcriptional regulatory region of an OCT3 gene" include, for example, mixtures prepared by adding DNAs to cell extracts included in commercially available *in vitro* transcription translation kits, wherein the added DNAs comprise structures such that a reporter gene is operably linked to a transcriptional regulatory region of an OCT3 gene.

In this method, "contact" can be achieved by adding test compounds to the culture media of "cells that comprise a DNA structured such that a reporter gene is operably linked to a transcriptional regulatory region of an OCT3 gene", or by adding test compounds to the above commercially available cell extracts, which comprise such DNAs. When the test compounds are proteins, the contact can also be achieved, for example, by introducing the cells with a DNA vector that expresses those proteins.

The next step in these methods is determining the level of reporter gene expression. The expression level of a reporter gene can be determined by methods that depend on the type of reporter gene, which are known to those skilled in the art. For example, when the reporter gene is the CAT gene, its expression level can be determined by detecting the acetylation of chloramphenicol, which is mediated by the CAT gene product. When the reporter gene is the lacZ gene, expression level can be determined by detecting color development in a chromogenic compound, mediated by the catalytic action of the lacZ gene expression product. When the reporter gene is a luciferase gene, the expression level can be determined by detecting the fluorescence of a fluorescent compound, mediated by the catalytic action of the luciferase gene expression product. Alternatively, when the reporter gene is the GFP gene, the expression level can be determined by detecting the fluorescence of the GFP protein.

In the present methods, compounds that decrease (suppress) or increase (enhance) the expression level of the reporter genes compared with that measured in the absence of the test compounds are selected. Compounds that cause a decrease (suppression) become agents for therapy of mental disorders, while compounds that cause an increase (enhancement) become agents for therapy of psychostimulant dependencies.

Another embodiment of the screening methods of the present invention uses the activity of organic cation transporter OCT3 protein as an indicator.

The above methods of the present invention are methods of screening for agents for therapy of mental disorders, comprising, for example, the following steps (a) to (c):
(a) contacting an organic cation transporter OCT3 protein or cells or cell extracts expressing said protein with a test compound;
(b) measuring the activity of said protein; and
(c) selecting a compound which decreases the activity of said protein compared with that measured in the absence of the test compounds.

Moreover, the above methods of the present invention are methods of screening for agents for therapy of psychostimulant dependencies, comprising, for example, the following steps (a) to (c):
(a) contacting an organic cation transporter OCT3 protein or cells or cell extracts expressing said protein with a test compound;
(b) measuring the activity of said protein; and
(c) selecting a compound which increases the activity of said protein compared with that measured in the absence of the test compounds.

In the present methods, the organic cation transporter OCT3 protein, or cells or cell extracts expressing said protein, are first contacted with test compounds.

Then, OCT3 protein activity is measured. OCT3 protein activities include, for example, the activity of transporting monoamines and their related agents. More specifically, the activities of transporting dopamines, serotonin, noradrenaline, dopamine neurotoxin MPP+, psychostimulants, and such are included. The activities can be measured by methods well known to those skilled in art.

For example, regulation of the activities of transporting the above monoamines and their related agents can be evaluated by labeling the above substances which can be transported by OCT3 with radioisotopes, then exposing these materials to test compounds and OCT3-expressing cells, and then comparing the radioactivity of the radiolabeled substances taken up by the cells after a certain period of the time. Even if radiolabeling is not possible, the activities of transporting the above monoamines and their related agents can be evaluated by measurement devices such as high performance liquid chromatography (HPLC) if the intracellular concentration of the substances is high enough.

Further, compounds that decrease (suppress) or increase (enhance) the activity of said proteins compared with that measured in the absence of the test compounds are selected. Compounds that decrease (suppress) the activity become agents for therapy of mental disorders, while compounds that increase (enhance) the activity become agents for therapy of psychostimulant dependencies.

The present invention further relates to methods of using the above animals of the present invention to identify compounds that cause mental disorders, and to screen for agents for the therapy of psychostimulant dependencies.

The methods of the present invention are methods for identifying compounds causing mental disorders, comprising the following steps (a) to (c):
(a) administering a test compound into a gene knockout non-human animal of the present invention;
(b) observing behaviors dependent on a phenotype of the above non-human animal; and
(c) determining that compounds which abolish the behaviors dependent on the above phenotypes cause a mental disorder.

The above methods of the present invention are methods for identifying compounds causing mental disorders, in which behaviors dependent on phenotypes of the animals of the present invention are used as indicators.

First, a test compound is administered into the above gene knockout non-human animals. Administration of the test compound can be either oral or parenteral; however, it is preferably parenteral administration, and specifically injection, transnasal administration, transpulmonary administration, or transdermal administration, etc. Examples of injection types are, for example, intravenous injections, intramuscular injections, intraperitoneal injections, and subcutaneous injections. Injections can be administered systemically or locally.

When DNAs are the test compounds, they can be administered into the body by viral vectors such as retroviruses, adenoviruses, and Sendai viruses and by non-viral vectors such as liposomes. Examples of administration methods include *in vivo* and *ex vivo* methods.

In the present methods, the behaviors dependent on the phenotypes of the OCT3 gene knockout non-human animals are then observed. The phenotypes of the OCT3 gene knockout non-human animals preferably indicate, for example, (a) anti-depression-like actions or (c) anti-anxiety actions.

Specifically, in the above methods, "the behaviors dependent on the above phenotypes are abolished" means, for example, the loss of the immobility condition in the forced swimming test and the persistence of exploratory behavior in a new place (ambulation, rearing behaviors, and such).

Furthermore, in the present invention, compounds that abolish the behaviors dependent on these phenotypes are selected. The selected compounds are determined to be compounds that cause mental disorders. These identified compounds which cause mental disorders are useful as reagents for elucidating the mechanisms of mental diseases.

Moreover, the methods of the present invention are methods for screening agents for therapy of psychostimulant dependencies, comprising the following steps (a) to (c):
(a) administering a test compound to gene knockout non-human animals of the present invention;
(b) observing the psychostimulant induced spontaneous locomotor activity dependent on the phenotype of the above non-human animals; and
(c) selecting compounds which abolish (decrease) the above locomotor activity.

Compounds selected in the above step (c) are determined to be agents for therapy of psychostimulant dependencies.

When using the agents or therapeutic compounds of the present invention as pharmaceutical agents, the agents and compounds themselves can be administered directly to patients, and they can also be administered as pharmaceutical compositions prepared by known pharmaceutical methods. For example, the drugs or compounds of the present invention can be formulated into forms suitable for oral or parenteral administration, such as tablets, pills, powders, granules, capsules, troches, syrups, liquids, emulsions, suspensions, injections (such as liquids and suspensions), suppositories, inhalants, percutaneous absorbents, eye drops, and eye ointments, by mixing with pharmacologically acceptable carriers (such as excipients, binders, disintegrators, flavors, corrigents, emulsifiers, diluents, and solubilizers).

The present invention also relates to methods for treating or preventing mental disorders which comprises administering a therapeutic agent for mental disorders or an anti-depressant action-enhancing agent of the present invention into individuals (for example, patients and the like). Further, the present invention also relates to methods for treating or preventing psychostimulant dependencies, which comprise administering a therapeutic agent for psychostimulant dependencies of the present invention into individuals (for example, patients and the like).

The individuals in the therapeutic methods of the present invention are generally patients with the above disorders and there in no particular limitation thereto; however, they are preferably humans.

Administration to patients can generally be carried out using methods known to those skilled in the art, such as intraarterial injection, intravenous injection, and subcutaneous injection. The administered doses vary depending on the weight and age of patients, the administration methods, or such, but those skilled in the art can appropriately select proper doses. Furthermore, if the compounds can be encoded by DNAs, the DNAs may be incorporated into vectors for gene therapy to perform gene therapy.

Examples of vectors for gene therapy include viral vectors such as retroviral vectors, adenoviral vectors, and adeno-associated virus vectors, as well as non-viral vectors such as liposomes. Using these vectors, DNAs of interest can be administered into patients by *ex vivo* methods, *in vivo* methods, and such.

Furthermore, the present invention relates to uses of substances suppressing the OCT3 expression or OCT3 function in the preparation of therapeutic agents for treating mental disorders or the preparation of anti-depressant action-enhancing agents.

All of the prior-art literature cited herein is incorporated herein by reference.

### Examples

Herein below, the present invention is specifically illustrated with reference to Examples, but it is not to be construed as being limited thereto.

### [Example 1] Effect of the regulation of OCT3 expression on depression-like symptoms

When experimental animals that has been made to swim in containers that prevented them from touching the bottom and from crawling out are made to swim again in the same container, they fall into despair and exhibit immobility for most of the experiment time. This condition of immobility is shortened by the administration of anti-depressants (example: chlordiazepoxide 100 mg/kg, imipramine 16 mg/kg), and therefore the present experiment (a forced swimming test) is often applied when screening for anti-depressants (Behav Brain Res. 73, 43-46, 1996). Thus, in this model the present inventors studied the effect of regulating OCT3 expression and its combined use with imipramine, an anti-depressant.

Male ddY mice were used for the experiments. Mice raised for three or more days after purchase were made to swim for 300 seconds in a beaker (15cm in diameter, 20cm in height). Immobility time was measured, and the mice were divided into groups such that the average immobility time was approximately the same for each group. The day after swimming an antisense (OCT3 antisense) constructed from an OCT3 gene sequence was continuously infused into the cerebral ventricle, using an osmotic pump according to the reported literature (J. Chem. Neuroanat. 2000. 20: 375-87). Further, a group infused with Ringer's solution, which is an antisense solvent (the solvent group) and a group infused with a cDNA sequence with the same bases as the antisense but in a random sequence without homology to known genes (the scrambled OCT3 antisense group) were also prepared. One week after infusion the mice were returned to the beakers and made to swim for 300 seconds, and immobility time was measured. Imipramine anti-depressant was intraperitoneally administered 30 minutes before the beginning of the test.

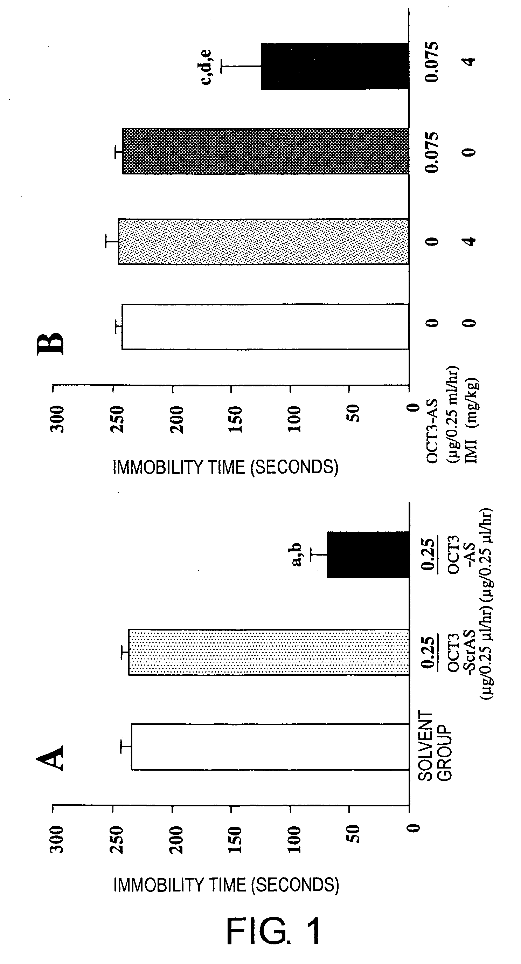

The results showed that in the solvent group depression-like symptoms appeared and the mice exhibit immobility for most of 300 seconds of swimming (average value: 234 ± 9 sec.) (Fig. 1A). Immobility was significantly reduced (average value: 69 ± 13 sec.) in the group in which mice were continuously infused with OCT3 antisense (0.25 µg/0.25 µl/hr). The scrambled OCT3 antisense group did not show any effect (average value: 236 ± 6 sec.) (Fig. 1A).

The effect when combined with anti-depressants was also studied. In the forced swimming test, no difference was observed between the solvent group (average value: 242 ± 4 sec.) and a low dose anti-depressant, imipramine (4 mg/kg), or a low dose OCT3 antisense (0.075 µg/0.25 µl/hr) [imipramine (4 mg/kg) average value: 245 ± 15 seconds; OCT3 antisense (0.075 µg/0.25 µl/hr) average value: 241 ± 6 sec.]. However, their combined use significantly reduced immobility (average value: 123 ± 33 sec.) (Fig. 1B).

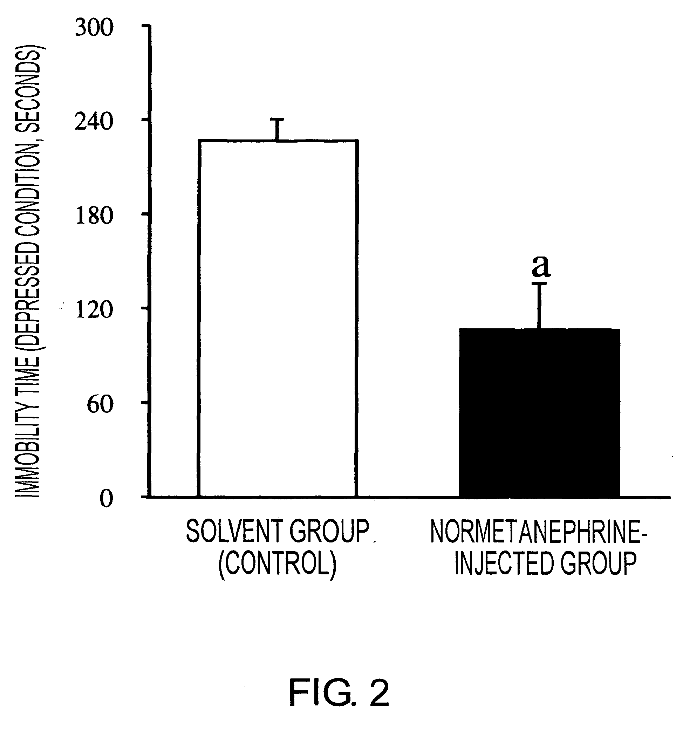

Next, the effect of normetanephrine, which has a high affinity for OCT3, was studied. Normetanephrine was continuously infused to the cerebral ventricle (2.5 µg/0.25 µl/hr) one day after the first forced swimming test, as with the antisense experiments described above. One week after the infusion, the mice were again made to swim in the beakers, and immobility time was measured. As a result, normetanephrine significantly reduced the immobility time (average value: 108 ± 29 sec.) compared with that of a solvent group (average value: 228 ± 13 sec.) (Fig. 2).

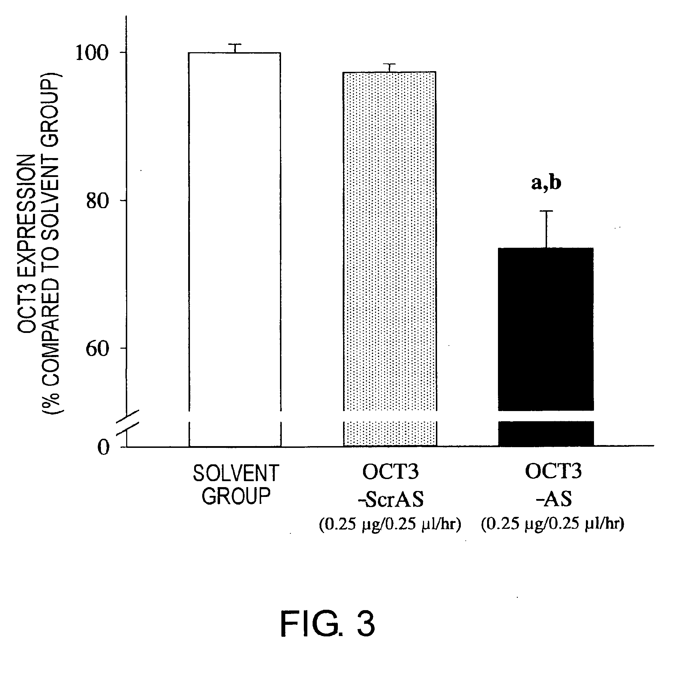

Further, intracerebroventricular infusion (one to two weeks) of OCT3 antisense reduced OCT3 expression in the brain by about 30 % compared with a pseudo-surgery group. However, this effect was about the same as that of antisense infusion into cerebral ventricle in the previous report (Fig. 3). OCT3 expression was not influenced in a scrambled OCT3 antisense group (Fig.3).

### [Example 2] The effect of OCT3 expression regulation on drug dependency-like symptoms

Continuous use of psychostimulants causes the development of dependency, and symptoms include increased sensitivity (reversed tolerance) to psychostimulants. The reversed tolerance phenomenon is sustained for long periods of time and reappears readily in spite of a long-term withdrawal after continuous use of psychostimulants. It cannot be treated with existing therapeutic drugs for mental disorders, and is therefore considered to be the result of irreversible changes in neuronal function. An increase in psychostimulant-dependent spontaneous locomotor activity is observed upon repeated administration of psychostimulants in experimental animals, and this phenomenon cannot be treated with existing therapeutic drugs for mental disorders. Therefore, increased psychostimulant-induced spontaneous locomotor activity caused by the repeated administration of psychostimulant in experimental animals is often used to analyze the mechanisms of drug dependency development. Thus, in the present model the present inventors studied the effect of OCT3 expression regulation.

Male ddY mice were used in the experiments. Mice raised for three or more days were divided into two groups. The cerebral ventricles of one group were continuously infused with antisense (OCT3 antisense), constructed from an OCT3 gene sequence, using a osmotic pump according to the reported literature (J. Chem. Neuroanat. 2000. 20: 375-87). Another group was subjected to pseudosurgery. Methamphetamine (1 mg/kg), a psychostimulant, was administered one week after the infusion, and psychostimulant-induced spontaneous locomotor activity was measured.

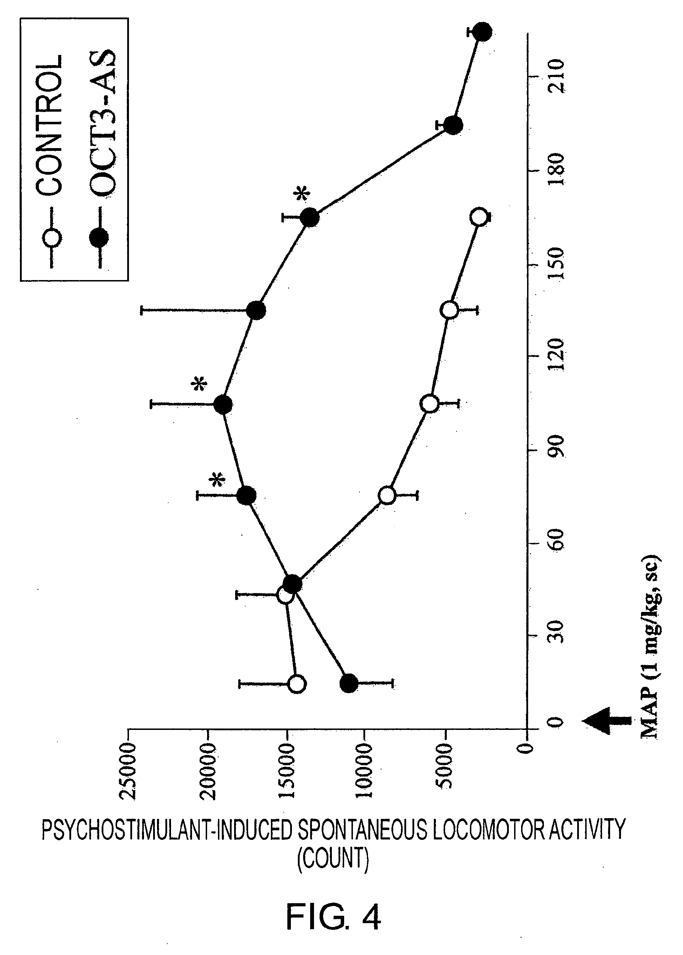

The results showed an increase in psychostimulant-induced spontaneous locomotor activity in mice infused with OCT3 antisense. That is, even a single administration of a psychostimulant showed the same behavior as the reversed tolerance phenomenon induced by repeated psychostimulant administration (Fig. 4). Furthermore, intracerebroventricular infusion (one to two weeks) of OCT3 antisense reduced OCT3 expression in the brain by about 30 % compared with a pseudo-surgery group. However, this effect was about the same as that of antisense infusion into cerebral ventricles in the previous report.

### [Example 3] The effect of OCT3 expression regulation on anxiety and exploratory behaviors in new places

Animals show exploratory behaviors (ambulation and rearing behaviors) when exposed to new, open spaces; however, such behaviors are reduced as time passes. Since such behaviors are diminished upon administration of anti-anxiety drugs and the animals maintain their exploratory behaviors, they can be used for screening anti-anxiety drugs. Therefore, in the present model the present inventors studied the effect of regulating OCT3 expression.

Male ddY mice were used in the experiments. Mice raised for three days or more were divided into two groups. The cerebral ventricles of one group were continuously infused with antisense (OCT3 antisense), constructed from an OCT3 gene sequence, using an osmotic pump according to the reported literature (J. Chem. Neuroanat. 2000. 20: 375-87). Another group was subjected to pseudosurgery. One week after the infusion the animals were placed in new large cages and the ambulation and rearing behavior were measured for 90 minutes.

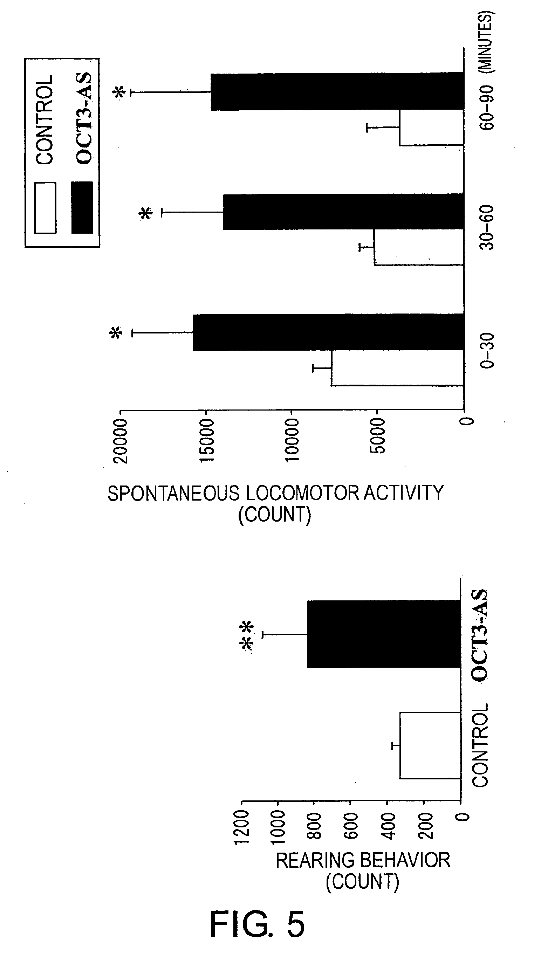

As a result, in the pseudosurgery group the ambulation decreased as time passed; however, mice infused with OCT3 antisense showed a significant increase in the ambulation immediately after they were placed in the cage, and this behavior was continued for 90 minutes (right side, Fig. 5). Further, a significant increase in rearing behavior, another sign of exploratory behavior, was also observed in mice infused with OCT3 antisense (left side, Fig 5). Furthermore, intracerebroventricular infusion (one to two weeks) of OCT3 antisense reduced OCT3 expression in the brain by about 30% compared with that of a pseudo-surgery group. However, this effect was about the same as that of antisense infusion into cerebral ventricles in the previous report.

### Industrial Applicability

The OCT3 gene knockout animals, constructed for the first time by the present inventors, show phenotypes related to mental disorders which are easily distinguished from those of wild type animals. Screening for compounds related to mental disorders such as depression, anxiety neuroses, and drug dependencies, or therapeutic agents for these mental disorders can be performed using said animals of the present invention. Compounds obtained by the screening methods of the present invention actually have the effect of changing phenotypes at the animal level, and are therefore expected to be highly effective agents.

Moreover, the above animals of the present invention are extremely useful as disease model animals for elucidating the mechanisms of each mental disorder. The mice with the anti-depression phenotype of the present invention show a striking anti-depression effect (for example, swimming for more than five minutes in a forced swimming test), and are therefore extremely useful as disease model animals.

Moreover, the present invention also demonstrated at the animal test level that substances that regulate OCT3 expression actually have a therapeutic effect on the above disorders. That is, regulating OCT3 expression by gene transfer using antisenses and vectors, and regulating OCT3 function using highly specific low molecule compounds is useful for treating the above disorders.

In addition to genetic factors, environmental factors such as stress are strongly suggested to be involved in mental disorders such as depression and anxiety, and thus even healthy individuals can develop such disorders on exposure to severe stress. Therefore, the inventors' finding that anti-anxiety and anti-depression effects were obtained in normal animals by reducing expression of target molecule OCT3 using antisense administration is an important discovery, and this valuable finding indicates how important OCT3 functional regulation is for depression and anxiety, which are greatly affected by environmental factors.

Further, the substances suppressing OCT3 expression of the present invention were found to have the effect of enhancing the activity of anti-depressants. Thus, said substances function as anti-depressant action-enhancing agents, and are extremely useful as concomitant drugs with existing anti-depressants. Anti-depressant activity can be promoted by the combined use of the anti-depressant action-enhancing agents of the present invention, even when the existing anti-depressants are at a dose that cannot show sufficient anti-depressant effect. For example, when a sufficient amount of an existing anti-depressant is difficult to administer because of side effects, a low dose that reduces said side effects can be used to obtain the desired effect by the combined use of an anti-depressant enhancing activity of the present invention.

## Claims

1. A therapeutic agent for a mental disorder comprising a substance suppressing the expression of an organic cation transporter OCT3 gene as an active ingredient.

2. The therapeutic agent of claim 1, wherein the substance suppressing the expression of an organic cation transporter OCT3 protein is a compound selected from the group consisting of:
(a) an antisense nucleic acid against a transcript of an OCT3 gene or a part thereof;
(b) a nucleic acid with a ribozyme activity which specifically cleaves a transcript of an OCT3 gene; and
(c) a nucleic acid with the action of inhibiting expression of an OCT3 gene by an RNAi effect.

3. A therapeutic agent for a mental disorder comprising a substance suppressing a function of an organic cation transporter OCT3 protein as an active ingredient.

4. The therapeutic agent of claim 3, wherein the substance suppressing a function of the organic cation transporter OCT3 protein is a compound of the following (a) or (b):
(a) an antibody that binds to the organic cation transporter OCT3 protein; and
(b) a small molecular compound that binds to the organic cation transporter OCT3 protein.

5. The therapeutic agent of any one of claims 1 to 4, wherein the mental disorder is depression or an anxiety neurosis.

6. A therapeutic agent for psychostimulant dependency, comprising a substance enhancing the expression or a function of an organic cation transporter OCT3 protein as an active ingredient.

7. A gene knockout non-human animal, in which expression of an organic cation transporter OCT3 gene is artificially suppressed.

8. The gene knockout non-human animal of claim 7, in which expression of the OCT3 gene is suppressed by the action of a nucleic acid of any one of:
(a) an antisense nucleic acid against a transcript of an OCT3 gene or a part thereof;
(b) a nucleic acid with a ribozyme activity which specifically cleaves an OCT3 gene transcript; and
(c) a nucleic acid with the action of inhibiting expression of an OCT3 gene by an RNAi effect.

9. The gene knockout non-human animal of claim 7, which shows a phenotype of any one of:
(a) an anti-depression-like action;
(b) an enhancement of psychostimulant-induced spontaneous locomotor activity; and
(c) an anti-anxiety action.

10. The gene knockout non-human animal of any one of claims 7 to 9, wherein the non-human animal is a rodent.

11. A method of screening for an agent for therapy of a mental disorder, comprising the steps of:
(a) contacting a test compound with an organic cation transporter OCT3 protein or its partial peptide;
(b) measuring the binding activity of the protein or its partial peptide with the test compound; and
(c) selecting a compound which binds to the organic cation transporter OCT3 protein or its partial peptide.

12. A method of screening for an agent for therapy of a mental disorder, comprising the steps of:
(a) contacting a test compound with a cell expressing an organic cation transporter OCT3 gene;
(b) measuring the expression level of the organic cation transporter OCT3 gene; and
(c) selecting a compound which reduces the expression level compared to that measured in the absence of the test compound.

13. A method of screening for an agent for therapy of a mental disorder, comprising the steps of:
(a) contacting a test compound with a cell comprising a DNA which comprises a structure in which a reporter gene is operably linked to a transcriptional control region of an organic cation transporter OCT3 gene;
(b) measuring the expression level of the reporter gene; and
(c) selecting a compound which reduces the expression level compared to that measured in the absence of the test compound.

14. A method of screening for an agent for therapy of a mental disorder, comprising the steps of:
(a) contacting a test compound with an organic cation transporter OCT3 protein or a cell or cell extract expressing the protein;
(b) measuring an activity of the protein; and
(c) selecting a compound which reduces the activity of the protein compared to that measured in the absence of the test compound.

15. A method for identifying a compound causing a mental disorder, comprising the steps of:
(a) administering a test compound to the gene knockout non-human animal of any one of claims 7 to 10;
(b) observing a behavior dependent on a phenotype of the non-human animal; and
(c) determining that a compound that abolishes the behavior dependent on the phenotype is a compound that causes a mental disorder.

16. The method of any one of claims 11 to 15, wherein the mental disorder is depression or an anxiety neurosis.

17. A method of screening for an agent for therapy of psychostimulant dependency, comprising the steps of:
(a) contacting a test compound with a cell expressing an organic cation transporter OCT3 gene;
(b) measuring the expression level of the organic cation transporter OCT3 gene; and
(c) selecting a compound which increases the expression level compared to that measured in the absence of the test compound.

18. A method of screening for an agent for therapy of psychostimulant dependency, comprising the steps of:
(a) contacting a test compound with a cell comprising a DNA which comprises a structure in which a reporter gene is operably linked to a transcriptional control region of an organic cation transporter OCT3 gene;
(b) measuring the expression level of the reporter gene; and
(c) selecting a compound which increases the expression level compared to that measured in the absence of the test compound.

19. A method of screening for an agent for therapy of psychostimulant dependency, comprising the steps of:
(a) contacting a test compound with an organic cation transporter OCT3 protein or a cell or cell extract expressing the protein;
(b) measuring an activity of the protein; and
(c) selecting a compound which increases the activity of the protein compared to that measured in the absence of the test compound.

20. A method of screening for an agent for therapy of psychostimulant dependency, comprising the steps of:
(a) administering a test compound to the gene knockout non-human animal of any one of claims 7 to 10;
(b) observing a psychostimulant-induced spontaneous locomotor activity that depends on a phenotype of the non-human animal; and
(c) selecting a compound that abolishes the locomotor activity.

21. A method for constructing the gene knockout non-human animal of claim 7, comprising a step of administering an antisense nucleic acid against a transcript of an OCT3 gene, or a part thereof, into the brain of a non-human animal.

22. An anti-depressant action enhancing agent comprising a substance suppressing the expression of an organic cation transporter OCT3 gene as an active ingredient.

23. The anti-depressant action enhancing agent of claim 22, wherein the substance suppressing the expression of the organic cation transporter OCT3 protein is a compound selected from the group consisting of:
(a) an antisense nucleic acid against a transcript of an OCT3 gene or a part thereof;
(b) a nucleic acid with a ribozyme activity which specifically cleaves a transcript of an OCT3 gene; and
(c) a nucleic acid with the action of inhibiting the expression of an OCT gene by an RNAi effect.

24. An anti-depressant action enhancing agent comprising a substance suppressing a function of an organic cation transporter OCT3 protein as an active ingredient.

25. The anti-depressant action enhancing agent of claim 24, wherein the substance suppressing the function of the organic cation transporter OCT3 protein is a compound of the following (a) or (b):
(a) an antibody which binds to the organic cation transporter OCT3 protein; and
(b) a small molecular compound which binds to the organic cation transporter OCT3 protein.

26. A pharmaceutical composition with an anti-depressant effect comprising an anti-depressant and the anti-depressant action enhancing agent of any one of claims 22 to 25 as active ingredients.
